# EUROPEAN PATENT SPECIFICATION

(11) **EP 4 313 233 B1**
(45) Date of publication and mention of the grant of the patent: **19.08.2026**
(21) Application number: 22778217.4
(22) Date of filing: 31.03.2022
(51) Int. Cl.: A61M 16/06, A61M 16/08

(54) **VENT FOR A RESPIRATORY SYSTEM**
ENTLÜFTUNG FÜR EIN ATEMSYSTEM
ÉVENT POUR UN SYSTÈME RESPIRATOIRE

(30) Priority: 31.03.2021 AU 2021900948
(43) Date of publication of application: 07.02.2024
(60) Divisional application: 26191244.8
(73) Proprietor: ResMed Pty Ltd, Bella Vista, New South Wales 2153 (AU)
(72) Inventor: YU, Hongjiang, Bella Vista, New South Wales 2153 (AU); KONZACK, Sabine, Bella Vista, New South Wales 2153 (AU); YUAN, Jie, Bella Vista, New South Wales 2153 (AU); FINLAY, Iain McNicol, Bella Vista, New South Wales 2153 (AU); KLINKENBERG, Luke Emmanuel, Bella Vista, New South Wales 2153 (AU)
(74) Representative: Vossius & Partner Patentanwälte Rechtsanwälte mbB
(86) International application number: PCT/AU2022/050293
(87) International publication number: WO 2022/204762

(56) References cited:
- WO-A1-2020/172708
- WO-A1-2020/172708
- WO-A1-2020/208523
- US-A1- 2018 185 597
- US-A1- 2019 262 568
- US-A1- 2019 262 568
- US-A1- 2020 061 330
- US-A1- 2020 179 640
- US-B2- 10 322 254
- US-B2- 8 397 727
- US-B2- 8 397 727
- US-B2- 9 480 809

## Description

### 2 BACKGROUND OF THE TECHNOLOGY

### 2.1 FIELD OF THE TECHNOLOGY

The present technology relates to one or more of the screening, diagnosis, monitoring, treatment, prevention and amelioration of respiratory-related disorders. The present technology also relates to medical devices or apparatus, and their use.

### 2.2 DESCRIPTION OF THE RELATED ART

US 2019/262568 A1 discloses a gas-washout vent that includes a housing with a first wall with one or more passages through the wall configured to provide fluid communication with a portion of the patient interface system exposed to a therapy pressure. The passages include respective first openings on a first surface of the first wall. The housing at least partially defines a second opening in communication with ambient atmosphere. A diffusing material is located at least partially within the housing to be adjacent the first surface. A surface of the diffusing material facing the first surface is spaced away from the first surface by a gap that extends to provide a fluid communication between all of the first openings and between all of the first openings and the second opening. The housing is configured so that air is prevented from flowing out of the housing at all areas directly opposite each of the first openings.

WO 2020/172708 A1 discloses: A vent structure may include a vent housing comprising an inlet, an outlet, at least one exhaust gas orifice, and a membrane having a first end and a second end spaced apart, the membrane having a moveable portion between the first end and the second end. The moveable portion may be spaced radially from a membrane-facing surface inside of the vent housing to form an exhaust gas flow passage therebetween to allow exhaust gas to flow from the pressurised volume through the exhaust gas flow passage to atmosphere via the exhaust gas orifice. The moveable portion may be elastically deformable and configured to move radially relative to the membrane-facing surface in response to pressure differences between an interior side of the membrane and an exterior side of the membrane to change a cross-sectional area of the exhaust gas flow passage and regulate the vent flow of gas.

US 8,397,727 B2 discloses: A vent arrangement is provided to a mask or associated conduit to discharge exhaled gas from the mask to atmosphere. The vent arrangement is structured to diffuse the exhaust vent flow to produce less air jetting, thereby increasing the comfort of the patient and their bed partner. For example, the vent arrangement may include one or more grill components and/or media constructed and arranged to diffuse vent flow.

### 2.2.1 Human Respiratory System and its Disorders

The respiratory system of the body facilitates gas exchange. The nose and mouth form the entrance to the airways of a patient.

The airways include a series of branching tubes, which become narrower, shorter and more numerous as they penetrate deeper into the lung. The prime function of the lung is gas exchange, allowing oxygen to move from the inhaled air into the venous blood and carbon dioxide to move in the opposite direction. The trachea divides into right and left main bronchi, which further divide eventually into terminal bronchioles. The bronchi make up the conducting airways, and do not take part in gas exchange. Further divisions of the airways lead to the respiratory bronchioles, and eventually to the alveoli. The alveolated region of the lung is where the gas exchange takes place, and is referred to as the respiratory zone. See *"*Respiratory Physiology", by John B. West, Lippincott Williams & Wilkins, 9th edition published 2012.

A range of respiratory disorders exist. Certain disorders may be characterised by particular events, e.g. apneas, hypopneas, and hyperpneas.

Examples of respiratory disorders include Obstructive Sleep Apnea (OSA), Cheyne-Stokes Respiration (CSR), respiratory insufficiency, Obesity Hyperventilation Syndrome (OHS), Chronic Obstructive Pulmonary Disease (COPD), Neuromuscular Disease (NMD) and Chest wall disorders.

Obstructive Sleep Apnea (OSA), a form of Sleep Disordered Breathing (SDB), is characterised by events including occlusion or obstruction of the upper air passage during sleep. It results from a combination of an abnormally small upper airway and the normal loss of muscle tone in the region of the tongue, soft palate and posterior oropharyngeal wall during sleep. The condition causes the affected patient to stop breathing for periods typically of 30 to 120 seconds in duration, sometimes 200 to 300 times per night. It often causes excessive daytime somnolence, and it may cause cardiovascular disease and brain damage. The syndrome is a common disorder, particularly in middle aged overweight males, although a person affected may have no awareness of the problem. See US Patent No. 4,944,310 (Sullivan).

Cheyne-Stokes Respiration (CSR) is another form of sleep disordered breathing. CSR is a disorder of a patient's respiratory controller in which there are rhythmic alternating periods of waxing and waning ventilation known as CSR cycles. CSR is characterised by repetitive de-oxygenation and re-oxygenation of the arterial blood. It is possible that CSR is harmful because of the repetitive hypoxia. In some patients CSR is associated with repetitive arousal from sleep, which causes severe sleep disruption, increased sympathetic activity, and increased afterload. See US Patent No. 6,532,959 (Berthon-Jones).

Respiratory failure is an umbrella term for respiratory disorders in which the lungs are unable to inspire sufficient oxygen or exhale sufficient CO₂ to meet the patient's needs. Respiratory failure may encompass some or all of the following disorders.

A patient with respiratory insufficiency (a form of respiratory failure) may experience abnormal shortness of breath on exercise.

Obesity Hyperventilation Syndrome (OHS) is defined as the combination of severe obesity and awake chronic hypercapnia, in the absence of other known causes for hypoventilation. Symptoms include dyspnea, morning headache and excessive daytime sleepiness.

Chronic Obstructive Pulmonary Disease (COPD) encompasses any of a group of lower airway diseases that have certain characteristics in common. These include increased resistance to air movement, extended expiratory phase of respiration, and loss of the normal elasticity of the lung. Examples of COPD are emphysema and chronic bronchitis. COPD is caused by chronic tobacco smoking (primary risk factor), occupational exposures, air pollution and genetic factors. Symptoms include: dyspnea on exertion, chronic cough and sputum production.

Neuromuscular Disease (NMD) is a broad term that encompasses many diseases and ailments that impair the functioning of the muscles either directly via intrinsic muscle pathology, or indirectly via nerve pathology. Some NMD patients are characterised by progressive muscular impairment leading to loss of ambulation, being wheelchair-bound, swallowing difficulties, respiratory muscle weakness and, eventually, death from respiratory failure. Neuromuscular disorders can be divided into rapidly progressive and slowly progressive: (i) Rapidly progressive disorders: Characterised by muscle impairment that worsens over months and results in death within a few years (e.g. Amyotrophic lateral sclerosis (ALS) and Duchenne muscular dystrophy (DMD) in teenagers); (ii) Variable or slowly progressive disorders: Characterised by muscle impairment that worsens over years and only mildly reduces life expectancy (e.g. Limb girdle, Facioscapulohumeral and Myotonic muscular dystrophy). Symptoms of respiratory failure in NMD include: increasing generalised weakness, dysphagia, dyspnea on exertion and at rest, fatigue, sleepiness, morning headache, and difficulties with concentration and mood changes.

Chest wall disorders are a group of thoracic deformities that result in inefficient coupling between the respiratory muscles and the thoracic cage. The disorders are usually characterised by a restrictive defect and share the potential of long term hypercapnic respiratory failure. Scoliosis and/or kyphoscoliosis may cause severe respiratory failure. Symptoms of respiratory failure include: dyspnea on exertion, peripheral oedema, orthopnea, repeated chest infections, morning headaches, fatigue, poor sleep quality and loss of appetite.

A range of therapies have been used to treat or ameliorate such conditions. Furthermore, otherwise healthy individuals may take advantage of such therapies to prevent respiratory disorders from arising. However, these have a number of shortcomings.

### 2.2.2 Therapies

Various respiratory therapies, such as Continuous Positive Airway Pressure (CPAP) therapy, Non-invasive ventilation (NIV), Invasive ventilation (IV), and High Flow Therapy (HFT) have been used to treat one or more of the above respiratory disorders.

### 2.2.2.1 Respiratory pressure therapies

Respiratory pressure therapy is the application of a supply of air to an entrance to the airways at a controlled target pressure that is nominally positive with respect to atmosphere throughout the patient's breathing cycle (in contrast to negative pressure therapies such as the tank ventilator or cuirass).

Continuous Positive Airway Pressure (CPAP) therapy has been used to treat Obstructive Sleep Apnea (OSA). The mechanism of action is that continuous positive airway pressure acts as a pneumatic splint and may prevent upper airway occlusion, such as by pushing the soft palate and tongue forward and away from the posterior oropharyngeal wall. Treatment of OSA by CPAP therapy may be voluntary, and hence patients may elect not to comply with therapy if they find devices used to provide such therapy one or more of: uncomfortable, difficult to use, expensive and aesthetically unappealing.

Non-invasive ventilation (NIV) provides ventilatory support to a patient through the upper airways to assist the patient breathing and/or maintain adequate oxygen levels in the body by doing some or all of the work of breathing. The ventilatory support is provided via a non-invasive patient interface. NIV has been used to treat CSR and respiratory failure, in forms such as OHS, COPD, NMD and Chest Wall disorders. In some forms, the comfort and effectiveness of these therapies may be improved.

Invasive ventilation (IV) provides ventilatory support to patients that are no longer able to effectively breathe themselves and may be provided using a tracheostomy tube. In some forms, the comfort and effectiveness of these therapies may be improved.

### 2.2.2.2 Flow therapies

Not all respiratory therapies aim to deliver a prescribed therapeutic pressure. Some respiratory therapies aim to deliver a prescribed respiratory volume, by delivering an inspiratory flow rate profile over a targeted duration, possibly superimposed on a positive baseline pressure. In other cases, the interface to the patient's airways is 'open' (unsealed) and the respiratory therapy may only supplement the patient's own spontaneous breathing with a flow of conditioned or enriched gas. In one example, High Flow therapy (HFT) is the provision of a continuous, heated, humidified flow of air to an entrance to the airway through an unsealed or open patient interface at a "treatment flow rate" that is held approximately constant throughout the respiratory cycle. The treatment flow rate is nominally set to exceed the patient's peak inspiratory flow rate. HFT has been used to treat OSA, CSR, respiratory failure, COPD, and other respiratory disorders. One mechanism of action is that the high flow rate of air at the airway entrance improves ventilation efficiency by flushing, or washing out, expired CO₂ from the patient's anatomical deadspace. Hence, HFT is thus sometimes referred to as a deadspace therapy (DST). Other benefits may include the elevated warmth and humidification (possibly of benefit in secretion management) and the potential for modest elevation of airway pressures. As an alternative to constant flow rate, the treatment flow rate may follow a profile that varies over the respiratory cycle.

Another form of flow therapy is long-term oxygen therapy (LTOT) or supplemental oxygen therapy. Doctors may prescribe a continuous flow of oxygen enriched gas at a specified oxygen concentration (from 21%, the oxygen fraction in ambient air, to 100%) at a specified flow rate (e.g., 1 litre per minute (LPM), 2 LPM, 3 LPM, etc.) to be delivered to the patient's airway.

### 2.2.3 Respiratory Therapy Systems

These respiratory therapies may be provided by a respiratory therapy system or device. Such systems and devices may also be used to screen, diagnose, or monitor a condition without treating it.

A respiratory therapy system may comprise a Respiratory Pressure Therapy Device (RPT device), an air circuit, a humidifier, a patient interface, an oxygen source, and data management.

### 2.2.3.1 Patient Interface

A patient interface may be used to interface respiratory equipment to its wearer, for example by providing a flow of air to an entrance to the airways. The flow of air may be provided via a mask to the nose and/or mouth, a tube to the mouth or a tracheostomy tube to the trachea of a patient. Depending upon the therapy to be applied, the patient interface may form a seal, e.g., with a region of the patient's face, to facilitate the delivery of gas at a pressure at sufficient variance with ambient pressure to effect therapy, e.g., at a positive pressure of about 10 cmH₂O relative to ambient pressure. For other forms of therapy, such as the delivery of oxygen, the patient interface may not include a seal sufficient to facilitate delivery to the airways of a supply of gas at a positive pressure of about 10 cmH₂O. For flow therapies such as nasal HFT, the patient interface is configured to insufflate the nares but specifically to avoid a complete seal. One example of such a patient interface is a nasal cannula.

Certain other mask systems may be functionally unsuitable for the present field. For example, purely ornamental masks may be unable to maintain a suitable pressure. Mask systems used for underwater swimming or diving may be configured to guard against ingress of water from an external higher pressure, but not to maintain air internally at a higher pressure than ambient.

Certain masks may be clinically unfavourable for the present technology e.g. if they block airflow via the nose and only allow it via the mouth.

Certain masks may be uncomfortable or impractical for the present technology if they require a patient to insert a portion of a mask structure in their mouth to create and maintain a seal via their lips.

Certain masks may be impractical for use while sleeping, e.g. for sleeping while lying on one's side in bed with a head on a pillow.

The design of a patient interface presents a number of challenges. The face has a complex three-dimensional shape. The size and shape of noses and heads varies considerably between individuals. Since the head includes bone, cartilage and soft tissue, different regions of the face respond differently to mechanical forces. The jaw or mandible may move relative to other bones of the skull. The whole head may move during the course of a period of respiratory therapy.

As a consequence of these challenges, some masks suffer from being one or more of obtrusive, aesthetically undesirable, costly, poorly fitting, difficult to use, and uncomfortable especially when worn for long periods of time or when a patient is unfamiliar with a system. Wrongly sized masks can give rise to reduced compliance, reduced comfort and poorer patient outcomes. Masks designed solely for aviators, masks designed as part of personal protection equipment (e.g. filter masks), SCUBA masks, or for the administration of anaesthetics may be tolerable for their original application, but nevertheless such masks may be undesirably uncomfortable to be worn for extended periods of time, e.g., several hours. This discomfort may lead to a reduction in patient compliance with therapy. This is even more so if the mask is to be worn during sleep.

CPAP therapy is highly effective to treat certain respiratory disorders, provided patients comply with therapy. If a mask is uncomfortable, or difficult to use a patient may not comply with therapy. Since it is often recommended that a patient regularly wash their mask, if a mask is difficult to clean (e.g., difficult to assemble or disassemble), patients may not clean their mask and this may impact on patient compliance.

While a mask for other applications (e.g. aviators) may not be suitable for use in treating sleep disordered breathing, a mask designed for use in treating sleep disordered breathing may be suitable for other applications.

For these reasons, patient interfaces for delivery of CPAP during sleep form a distinct field.

### 2.2.3.2 Respiratory Pressure Therapy (RPT) Device

A respiratory pressure therapy (RPT) device may be used individually or as part of a system to deliver one or more of a number of therapies described above, such as by operating the device to generate a flow of air for delivery to an interface to the airways. The flow of air may be pressure-controlled (for respiratory pressure therapies) or flow-controlled (for flow therapies such as HFT). Thus RPT devices may also act as flow therapy devices. Examples of RPT devices include a CPAP device and a ventilator.

### 2.2.3.3 Air circuit

An air circuit is a conduit or a tube constructed and arranged to allow, in use, a flow of air to travel between two components of a respiratory therapy system such as the RPT device and the patient interface. In some cases, there may be separate limbs of the air circuit for inhalation and exhalation. In other cases, a single limb air circuit is used for both inhalation and exhalation.

### 2.2.3.4 Humidifier

Delivery of a flow of air without humidification may cause drying of airways. The use of a humidifier with an RPT device and the patient interface produces humidified gas that minimizes drying of the nasal mucosa and increases patient airway comfort. In addition in cooler climates, warm air applied generally to the face area in and about the patient interface is more comfortable than cold air. Humidifiers therefore often have the capacity to heat the flow of air was well as humidifying it.

### 2.2.3.5 Vent technologies

Some forms of treatment systems may include a vent to allow the washout of exhaled carbon dioxide. The vent may allow a flow of gas from an interior space of a patient interface, e.g., the plenum chamber, to an exterior of the patient interface, e.g., to ambient.

The vent may comprise an orifice and gas may flow through the orifice in use of the mask. Many such vents are noisy. Others may become blocked in use and thus provide insufficient washout. Some vents may be disruptive of the sleep of a bed partner 1100 of the patient 1000, e.g. through noise or focussed airflow.

ResMed Limited has developed a number of improved mask vent technologies. See International Patent Application Publication No. WO 1998/034,665; International Patent Application Publication No. WO 2000/078,381; US Patent No. 6,581,594; US Patent Application Publication No. US 2009/0050156; US Patent Application Publication No. 2009/0044808.

Table of noise of prior masks (ISO 17510-2:2007, 10 cmH₂O pressure at 1m)

| Mask name | Mask type | A-weighted sound power level dB(A) (uncertainty) | A-weighted sound pressure dB(A) (uncertainty) | Year (approx.) |
|---|---|---|---|---|
| Glue-on (*) | nasal | 50.9 | 42.9 | 1981 |
| ResCare standard (*) | nasal | 31.5 | 23.5 | 1993 |
| ResMed Mirage^{™} (*) | nasal | 29.5 | 21.5 | 1998 |
| ResMed UltraMirage^{™} | nasal | 36 (3) | 28 (3) | 2000 |
| ResMed Mirage Activa^{™} | nasal | 32 (3) | 24 (3) | 2002 |
| ResMed Mirage Micro^{™} | nasal | 30 (3) | 22 (3) | 2008 |
| ResMed Mirage^{™} SoftGel | nasal | 29 (3) | 22 (3) | 2008 |
| ResMed Mirage^{™} FX | nasal | 26 (3) | 18 (3) | 2010 |
| ResMed Mirage Swift^{™} (*) | nasal pillows | 37 | 29 | 2004 |
| ResMed Mirage Swift^{™} II | nasal pillows | 28 (3) | 20 (3) | 2005 |
| ResMed Mirage Swift^{™} LT | nasal pillows | 25 (3) | 17 (3) | 2008 |
| ResMed AirFit P10 | nasal pillows | 21 (3) | 13 (3) | 2014 |

| | | | | |
|---|---|---|---|---|
| (* one specimen only, measured using test method specified in ISO 3744 in CPAP mode at 10 cmH₂O) | | | | |

Sound pressure values of a variety of objects are listed below

| Object | A-weighted sound pressure dB(A) | Notes |
|---|---|---|
| Vacuum cleaner: Nilfisk | 68 | ISO 3744 at 1m distance |
| Walter Broadly Litter Hog: B+ Grade | | |
| Conversational speech | 60 | 1m distance |
| Average home | 50 | |
| Quiet library | 40 | |
| Quiet bedroom at night | 30 | |
| Background in TV studio | 20 | |

### 3 BRIEF SUMMARY OF THE TECHNOLOGY

The present technology is directed towards providing medical devices used in the screening, diagnosis, monitoring, amelioration, treatment, or prevention of respiratory disorders having one or more of improved comfort, cost, efficacy, ease of use and manufacturability.

A first aspect of the present technology relates to apparatus used in the screening, diagnosis, monitoring, amelioration, treatment or prevention of a respiratory disorder.

Another aspect of the present technology relates to methods used in the screening, diagnosis, monitoring, amelioration, treatment or prevention of a respiratory disorder.

An aspect of certain forms of the present technology is to provide methods and/or apparatus that improve the compliance of patients with respiratory therapy.

An aspect of the present technology relates to a vent structure for a respiratory therapy system. In certain forms, the vent structure may form part of a connection member for a respiratory therapy system. In certain forms, the vent structure may form part of a patient interface for a respiratory therapy system.

One form of the present technology comprises a vent structure for a respiratory therapy system, the vent structure comprising a vent housing defining a flow path for a flow of air being vented from the respiratory therapy system, a vent inlet configured to allow the flow of air to enter the flow path, and a vent outlet configured to allow the flow of air to exit the flow path into the surrounding ambient air, wherein the vent housing is configured so that the flow path comprises a curved turning region in which the flow path changes direction.

In certain forms, the vent housing is configured to comprise a curved turning region defining a curved flow path in which the flow path changes direction by at least 90°. In certain forms, the vent housing is configured so that, through the turning region, the flow path changes direction to a substantially opposite direction. According to the invention the vent housing is configured so that the flow path comprises a curved turning region in which the flow path changes direction by an angle of substantially 180°.

In certain forms, the width of the flow path at the turning region and at a region downstream of the turning region is of a size that is sufficiently large to avoid water droplets in the flow of air blocking the flow path while being sufficiently small to regulate the flow rate. For example, in certain forms, the width of the flow path at the turning region and at a region downstream of the turning region is at least substantially 0.85mm. In certain forms, the width of the flow path is in the range 0.75mm - 1.5mm.

One form of the present technology comprises a vent structure for a respiratory therapy system, the vent structure comprising a vent housing defining a flow path for a flow of air being vented from the respiratory therapy system, a vent inlet configured to allow the flow of air to enter the flow path, and a vent outlet configured to allow the flow of air to exit the flow path into the surrounding ambient air, wherein the vent housing is configured so that the flow path comprises a curved turning region in which the flow path changes direction by at least 90°, wherein the width of the flow path at the turning region and at a region downstream of the turning region is at least substantially 0.85mm.

One form of the present technology comprises a vent structure for a respiratory therapy system, the vent structure comprising a vent housing defining a flow path for a flow of air being vented from the respiratory therapy system, a vent inlet configured to allow the flow of air to enter the flow path, and a vent outlet configured to allow the flow of air to exit the flow path into the surrounding ambient air, wherein the vent housing is configured so that the flow path comprises a curved turning region in which the flow path changes direction, and wherein the vent housing comprises an inner path surface on an inner side of the flow path and an outer path surface on an outer side of the flow path. In certain forms, an opening angle between the inner path surface of the vent housing at the vent outlet and the outer path surface of the vent housing at the vent outlet is substantially 7° or less.

In examples: a) in the turning region, the inner path surface has a curved cross-sectional shape, for example in the form of a segment of a circle; b) the inner path surface has a cross-sectional shape in, or resembling, the form of part of an aerofoil; c) the aerofoil defines a chord line having a chord length of at least 50mm; d) a region of maximum thickness of the aerofoil is located along the chord line at a distance from an end of the chord line proximate the turning region that is at least 25% of the chord length; e) the vent outlet is located downstream of the region of maximum thickness; and/or f) the vent housing comprises a plurality of partitions which form a plurality of flow paths therebetween, wherein the plurality of flow paths comprise the flow path and the other flow paths of the plurality of flow paths are similar to the flow path.

One form of the present technology comprises a patient interface comprising a plenum chamber pressurisable to a therapeutic pressure of at least 6 cmH₂O above ambient air pressure, said plenum chamber including a plenum chamber inlet port sized and structured to receive a flow of air at the therapeutic pressure for breathing by a patient, a seal-forming structure constructed and arranged to form a seal with a region of the patient's face surrounding an entrance to the patient's airways, said seal-forming structure having a hole therein such that the flow of air at said therapeutic pressure is delivered to at least an entrance to the patient's nares, the seal-forming structure constructed and arranged to maintain said therapeutic pressure in the plenum chamber throughout the patient's respiratory cycle in use, and a vent structure according to any one of the forms of the technology described above, wherein the vent structure allows a continuous flow of gases exhaled by the patient from an interior of the plenum chamber to ambient, said vent structure being sized and shaped to maintain the therapeutic pressure in the plenum chamber in use, and wherein the patient interface is configured to allow the patient to breath from ambient through their mouth in the absence of a flow of pressurised air through the plenum chamber inlet port, or the patient interface is configured to leave the patient's mouth uncovered.

One form of the present technology comprises a connection member configured to directly or indirectly fluidly connect an air circuit to a patient interface in a respiratory therapy system, the connection member comprising a tube portion comprising a first end configured to directly or indirectly fluidly connect to the air circuit and a second end configured to directly or indirectly fluidly connect to the patient interface, and a vent structure according to any one of the forms of the technology described above, the vent structure being configured to allow air in the tube portion to exit into the surrounding ambient air.

In examples: a) the tube portion comprises an outer tube portion and an inner tube portion; b) the outer tube portion is rotatable relative to the inner tube portion around a mutual longitudinal axis; and/or c) the outer tube portion comprises the first end and the inner tube portion comprises the second end.

One form of the present technology comprises a connection member configured to directly or indirectly fluidly connect an air circuit to a patient interface in a respiratory system, the connection member comprising a tube portion configured to fluidly connect the air circuit to the patient interface, the tube portion comprising a first end configured to directly or indirectly fluidly connect to the air circuit, a second end configured to directly or indirectly fluidly connect to the patient interface, an outer tube portion, and an inner tube portion, wherein the outer tube portion is rotatable relative to the inner tube portion and outer tube portion around a mutual longitudinal axis, wherein the connection member further comprises a vent structure for venting air inside the inner tube portion to exit into the surrounding ambient air, the vent structure comprising a vent housing comprising at least a portion of the outer tube portion, wherein the outer tube portion solely defines at least a part of a flow path for a flow of air being vented and wherein the vent housing defines a vent inlet configured to allow the flow of air to enter the flow path from inside the connection member, and a vent outlet configured to allow the flow of air to exit the vent housing into surrounding ambient air, wherein the vent structure further comprises a noise attenuation structure for reducing noise generated by a flow of air between the outer tube portion and the inner tube portion.

In examples: a) the flow path is substantially parallel to the mutual longitudinal axis; b) the noise attenuation structure comprises portions of the inner tube portion and/or the outer tube portion configured to form a stepped region in a gap between the inner tube portion and the outer tube portion; c) the noise attenuation structure comprises portions of the inner tube portion and/or the outer tube portion configured to form an acoustic attenuation chamber in a gap between the inner tube portion and the outer tube portion; d) the part of the flow path defined by the outer tube portion is a first flow path portion, wherein the vent housing further comprises a portion of the inner tube portion, wherein the inner tube portion and the outer tube portion define therebetween a second flow path portion, wherein the flow path comprises the second flow path portion and wherein the second flow path portion receives the flow of air from the first flow path portion and delivers the flow of air to the vent outlet; e) the second flow path portion comprises a curved turning region in which the flow path changes direction by at least 90°; f) through the turning region the flow path changes direction to a substantially opposite direction; g) through the turning region the flow path changes direction by an angle of substantially 180°; h) the first end is configured to directly fluidly connect to a first portion of the air circuit and the second end is configured to directly fluidly connect to a second portion of the air circuit; i) the outer tube portion comprises the first end and the inner tube portion comprises the second end; j) the connection member comprises a flow inhibitor to inhibit air inside the inner tube portion from exiting into the surrounding ambient air by flowing between the inner tube portion and the outer tube portion; and/or k) the flow inhibitor comprises an annular seal positioned to substantially block the flow of air through an opening to a gap between the inner tube portion and the outer tube portion.

One form of the present technology comprises a connection member configured to directly or indirectly fluidly connect an air circuit to a patient interface in a respiratory therapy system, the connection member comprising a tube portion configured to fluidly connect the air circuit to the patient interface, the tube portion comprising a first end configured to directly or indirectly fluidly connect to the air circuit, and a second end configured to directly or indirectly fluidly connect to the patient interface, wherein the connection member further comprises a vent structure for venting air from an interior volume of the tube portion, the vent structure comprising a vent housing comprising at least a portion of the tube portion, and a plurality of partitions inside the vent housing, the plurality of partitions forming therebetween a plurality of flow paths, wherein the flow paths each comprise a vent inlet configured to receive an air flow, and a vent outlet configured to allow the air flow to exit into the surrounding ambient air, wherein the flow paths are curved around the tube portion.

In examples: a) the vent inlet and the vent outlet are circumferentially offset around the tube portion; b) each of the flow paths form a spiral shape around the tube portion; c) the vent inlet is positioned closer to the first end of the tube portion; d) the vent outlet is positioned closer to the second end of the tube portion; e) the tube portion comprises an outer tube portion, and an inner tube portion; f) the outer tube portion is rotatable relative to the inner tube portion around a mutual longitudinal axis; g) the vent housing comprises at least a portion of the outer tube portion; h) the vent housing comprises at least a portion of the inner tube portion; i) the partitions are formed on an outer surface of the inner tube portion; j) the first end is configured to directly fluidly connect to a first portion of the air circuit and the second end is configured to directly fluidly connect to a second portion of the air circuit; k) a mushroom-shaped portion includes a turning region configured to change the direction of air flow to a substantially opposite direction; and/or I) through the turning region the air flow changes direction by an angle of substantially 180°.

An aspect of one form of the present technology is a method of manufacturing apparatus.

An aspect of certain forms of the present technology is a medical device that is easy to use, e.g. by a person who does not have medical training, by a person who has limited dexterity, vision or by a person with limited experience in using this type of medical device.

An aspect of one form of the present technology is a portable RPT device that may be carried by a person, e.g., around the home of the person.

An aspect of one form of the present technology is a patient interface that may be washed in a home of a patient, e.g., in soapy water, without requiring specialised cleaning equipment. An aspect of one form of the present technology is a humidifier tank that may be washed in a home of a patient, e.g., in soapy water, without requiring specialised cleaning equipment.

The methods, systems, devices and apparatus described may be implemented so as to improve the functionality of a processor, such as a processor of a specific purpose computer, respiratory monitor and/or a respiratory therapy apparatus. Moreover, the described methods, systems, devices and apparatus can provide improvements in the technological field of automated management, monitoring and/or treatment of respiratory conditions, including, for example, sleep disordered breathing.

Of course, portions of the aspects may form sub-aspects of the present technology. Also, various ones of the sub-aspects and/or aspects may be combined in various manners and also constitute additional aspects or sub-aspects of the present technology.

Other features of the technology will be apparent from consideration of the information contained in the following detailed description, abstract, drawings and claims.

### 4 BRIEF DESCRIPTION OF THE DRAWINGS

The present technology is illustrated by way of example, and not by way of limitation, in the figures of the accompanying drawings, in which like reference numerals refer to similar elements including:

### 4.1 RESPIRATORY THERAPY SYSTEMS

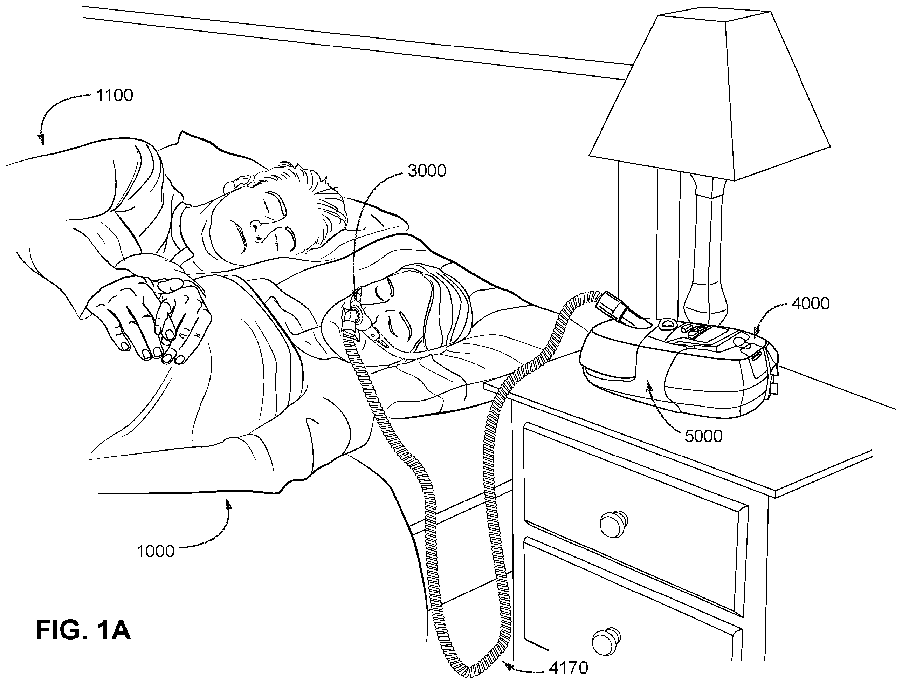

Fig. 1A shows a system including a patient 1000 wearing a patient interface 3000, in the form of nasal pillows, receiving a supply of air at positive pressure from an RPT device 4000. Air from the RPT device 4000 is conditioned in a humidifier 5000, and passes along an air circuit 4170 to the patient 1000. A bed partner 1100 is also shown. The patient is sleeping in a supine sleeping position.

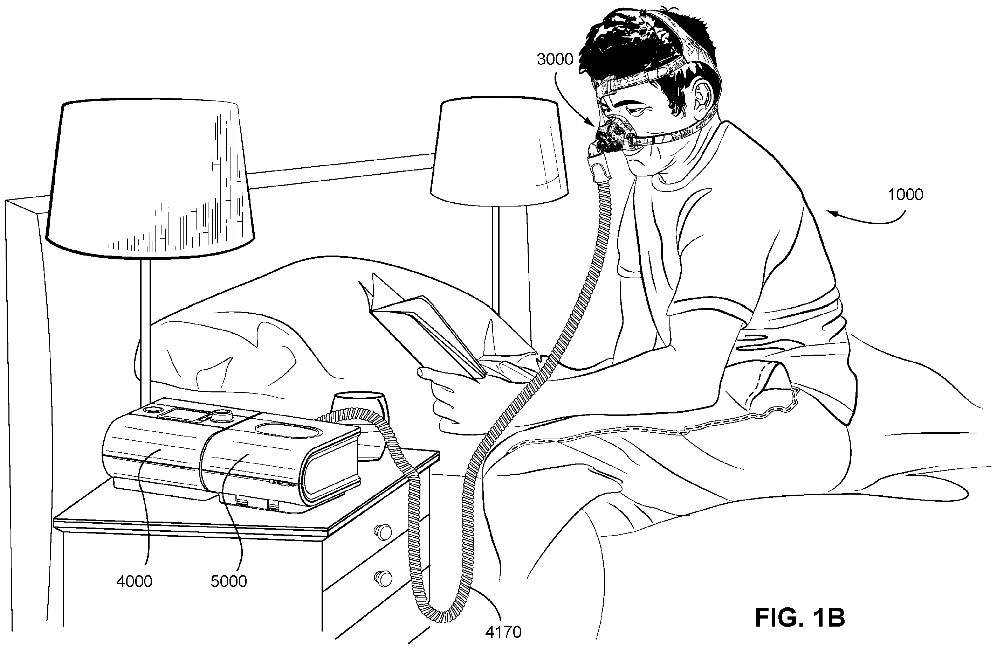

Fig. 1B shows a system including a patient 1000 wearing a patient interface 3000, in the form of a nasal mask, receiving a supply of air at positive pressure from an RPT device 4000. Air from the RPT device is humidified in a humidifier 5000, and passes along an air circuit 4170 to the patient 1000.

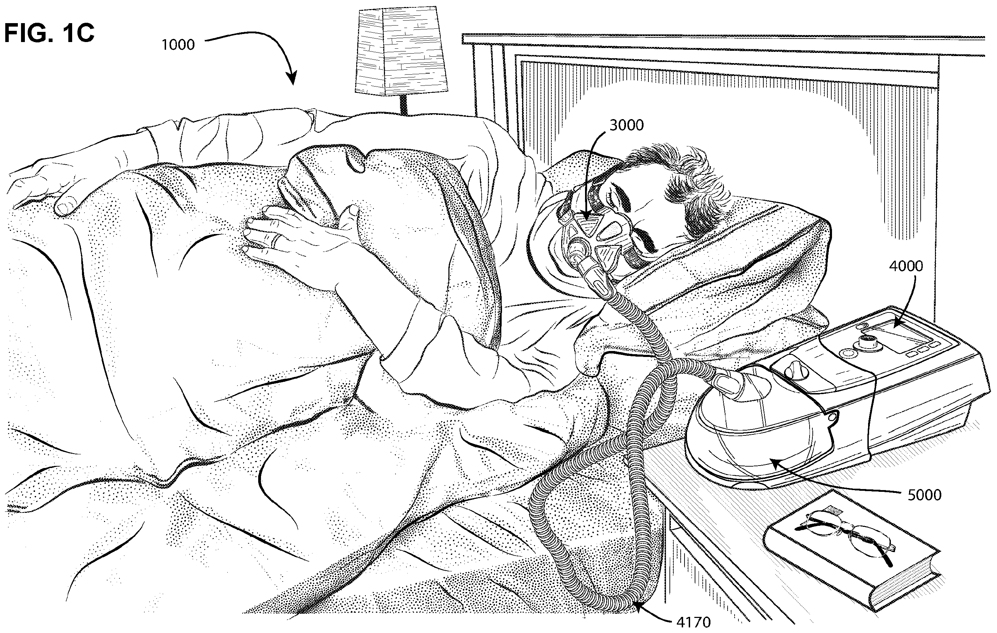

Fig. 1C shows a system including a patient 1000 wearing a patient interface 3000, in the form of a full-face mask, receiving a supply of air at positive pressure from an RPT device 4000. Air from the RPT device is humidified in a humidifier 5000, and passes along an air circuit 4170 to the patient 1000. The patient is sleeping in a side sleeping position.

### 4.2 RESPIRATORY SYSTEM AND FACIAL ANATOMY

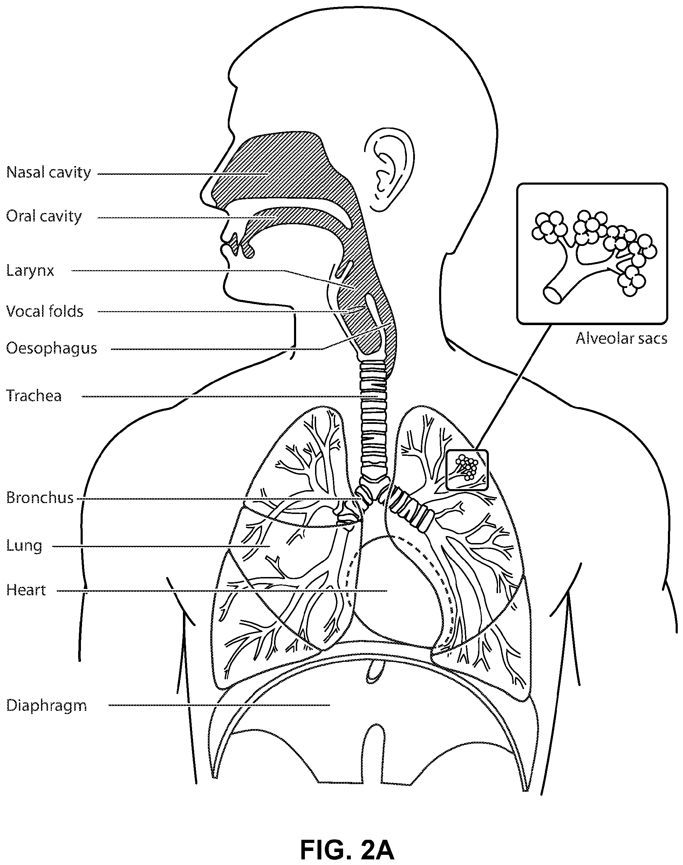

Fig. 2A shows an overview of a human respiratory system including the nasal and oral cavities, the larynx, vocal folds, oesophagus, trachea, bronchus, lung, alveolar sacs, heart and diaphragm.

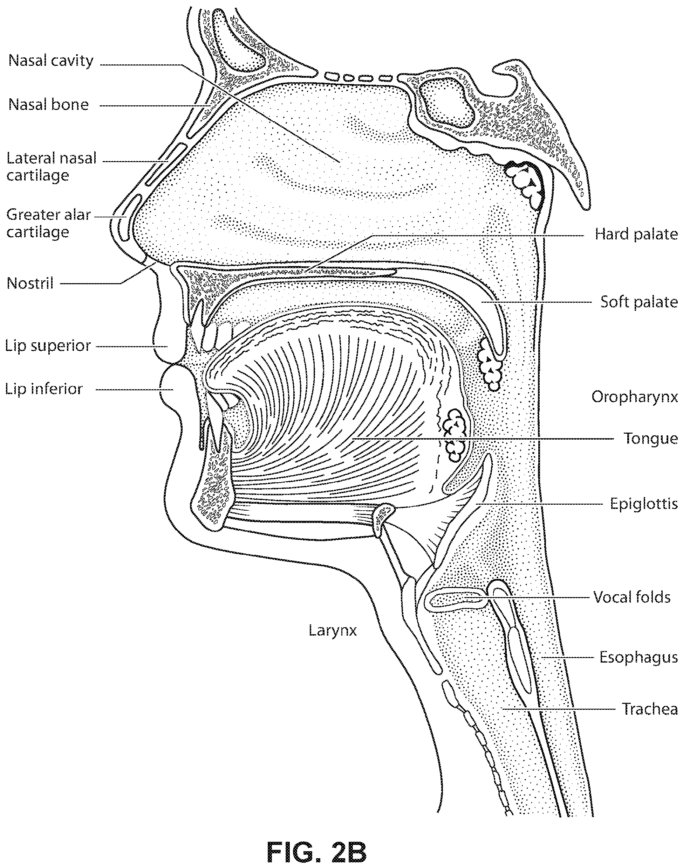

Fig. 2B shows a view of a human upper airway including the nasal cavity, nasal bone, lateral nasal cartilage, greater alar cartilage, nostril, lip superior, lip inferior, larynx, hard palate, soft palate, oropharynx, tongue, epiglottis, vocal folds, oesophagus and trachea.

### 4.3 PATIENT INTERFACE

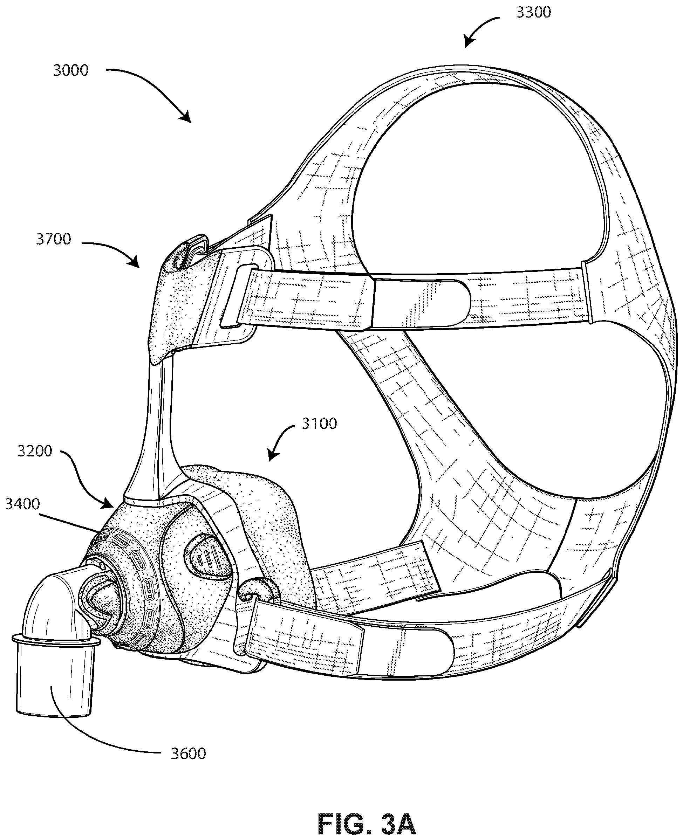

Fig. 3A shows a patient interface in the form of a nasal mask in accordance with one form of the present technology.

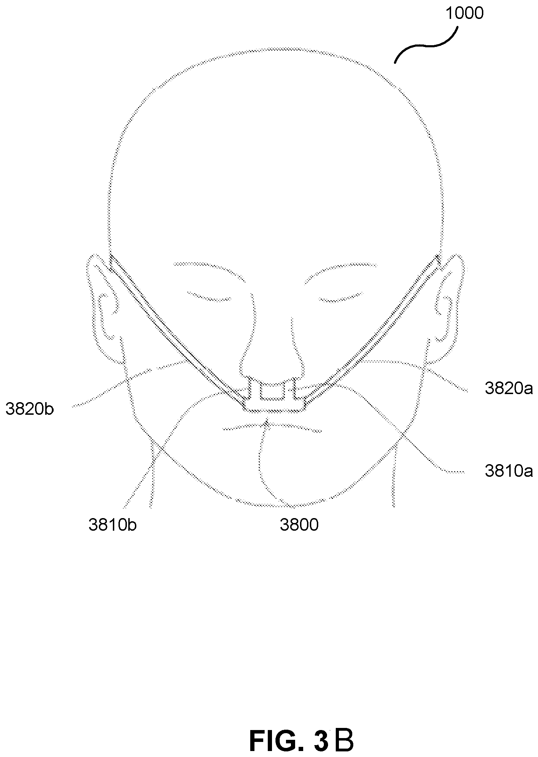

Fig. 3B shows a patient interface in the form of a nasal cannula in accordance with one form of the present technology.

### 4.4 RPT DEVICE

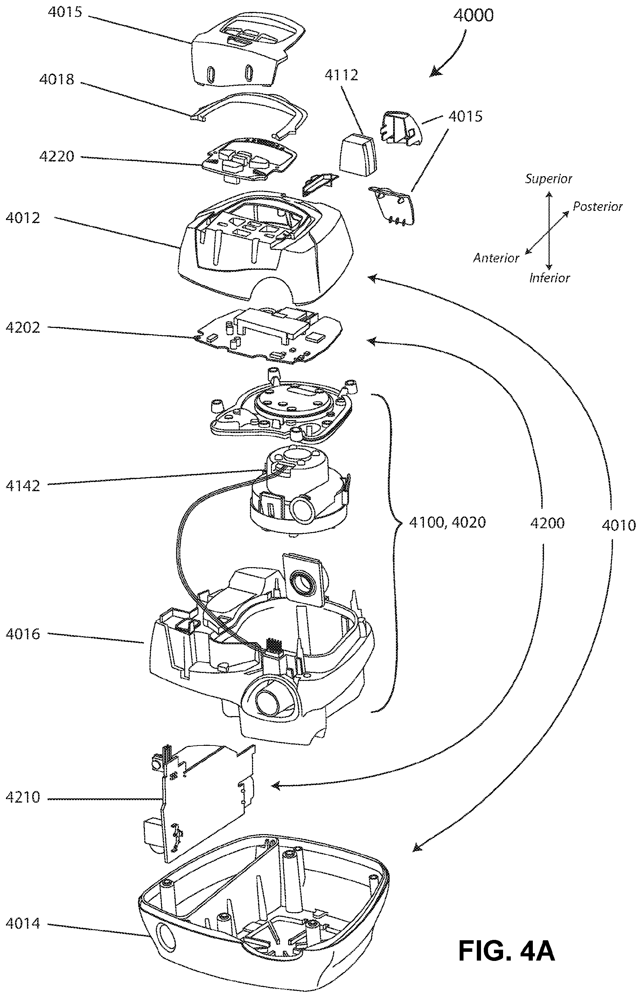

Fig. 4A shows an RPT device in accordance with one form of the present technology.

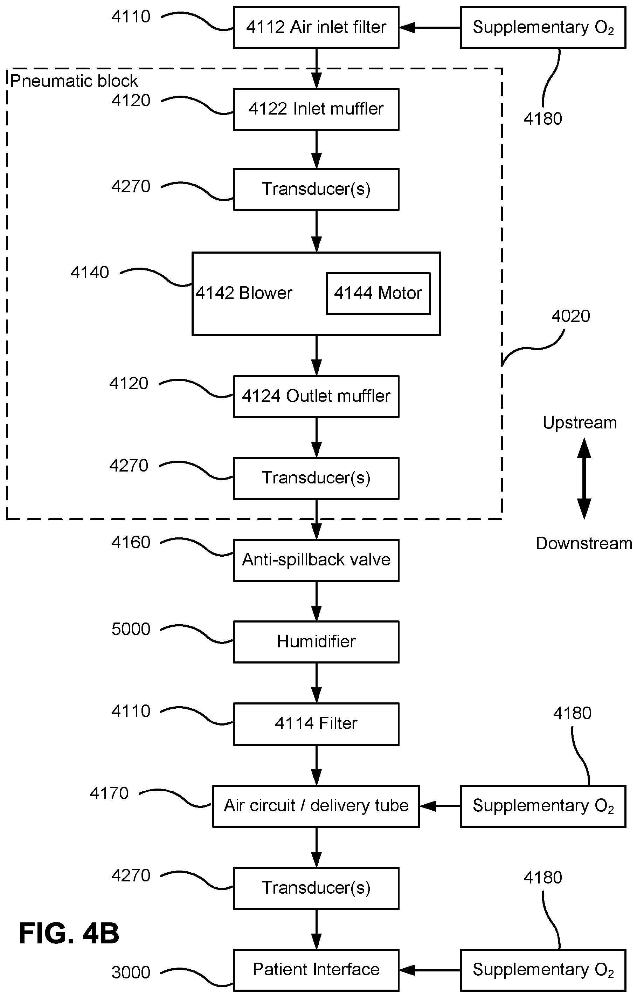

Fig. 4B is a schematic diagram of the pneumatic path of an RPT device in accordance with one form of the present technology. The directions of upstream and downstream are indicated with reference to the blower and the patient interface. The blower is defined to be upstream of the patient interface and the patient interface is defined to be downstream of the blower, regardless of the actual flow direction at any particular moment. Items which are located within the pneumatic path between the blower and the patient interface are downstream of the blower and upstream of the patient interface.

### 4.5 HUMIDIFIER

Fig. 5A shows an isometric view of a humidifier in accordance with one form of the present technology.
Fig. 5B shows an isometric view of a humidifier in accordance with one form of the present technology, showing a humidifier reservoir 5110 removed from the humidifier reservoir dock 5130.

### 4.6 BREATHING WAVEFORMS

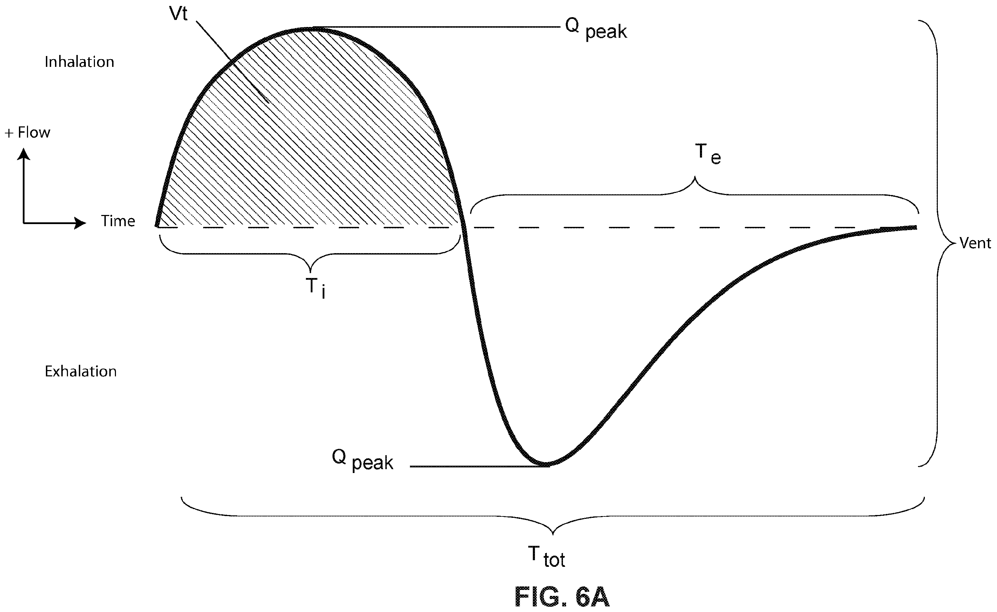

Fig. 6A shows a model typical breath waveform of a person while sleeping.

### 4.7 VENT

Fig. 7A shows a perspective view of a connection member according to one form of the present technology.
Fig. 7B shows an exploded perspective view of the connection member of Fig. 7A.
Fig. 7C shows a top view of the connection member of Fig 7A.
Fig. 7D shows a bottom view of the connection member of Fig 7A.
Fig. 7E shows a side view of the connection member of Fig 7A. The side view of the connection member is the same from all sides.
Fig. 7F shows a cross-sectional view along the plane A-A of Fig. 7E.
Fig. 8A shows a front view of a mask with two vent structures according to one form of the present technology.
Fig. 8B shows a bottom cross-sectional view of region G indicated in Fig. 8A along the line H.
Fig. 8C shows a cross-sectional view of a portion of a mask including a vent structure according to one form of the present technology.
Fig. 8D shows a cross-sectional view of a portion of a mask including a vent structure according to one form of the present technology.
Fig. 9A shows an exploded perspective view of a connection member according to one form of the present technology.

### 5 DETAILED DESCRIPTION OF EXAMPLES OF THE TECHNOLOGY

Before the present technology is described in further detail, it is to be understood that the technology is not limited to the particular examples described herein, which may vary. It is also to be understood that the terminology used in this disclosure is for the purpose of describing only the particular examples discussed herein, and is not intended to be limiting.

The following description is provided in relation to various examples which may share one or more common characteristics and/or features.

### 5.1 THERAPY

In one form, the present technology comprises a method for treating a respiratory disorder comprising applying positive pressure to the entrance of the airways of a patient 1000.

In certain examples of the present technology, a supply of air at positive pressure is provided to the nasal passages of the patient via one or both nares.

In certain examples of the present technology, mouth breathing is limited, restricted or prevented.

### 5.2 RESPIRATORY THERAPY SYSTEMS

In one form, the present technology comprises a respiratory therapy system for treating a respiratory disorder. The respiratory therapy system may comprise an RPT device 4000 for supplying a flow of air to the patient 1000 via an air circuit 4170 and a patient interface 3000 or 3800.

### 5.3 PATIENT INTERFACE

A non-invasive patient interface 3000 in accordance with one aspect of the present technology comprises the following functional aspects: a seal-forming structure 3100, a plenum chamber 3200, a positioning and stabilising structure 3300, a vent 3400, one form of connection port 3600 for connection to air circuit 4170, and a forehead support 3700. In some forms a functional aspect may be provided by one or more physical components. In some forms, one physical component may provide one or more functional aspects. In use the seal-forming structure 3100 is arranged to surround an entrance to the airways of the patient so as to maintain positive pressure at the entrance(s) to the airways of the patient 1000. The sealed patient interface 3000 is therefore suitable for delivery of positive pressure therapy.

An unsealed patient interface 3800, in the form of a nasal cannula, includes nasal prongs 3810a, 3810b which can deliver air to respective nares of the patient 1000 via respective orifices in their tips. Such nasal prongs do not generally form a seal with the inner or outer skin surface of the nares. The air to the nasal prongs may be delivered by one or more air supply lumens 3820a, 3820b that are coupled with the nasal cannula 3800. The lumens 3820a, 3820b lead from the nasal cannula 3800 to a respiratory therapy device via an air circuit. The unsealed patient interface 3800 is particularly suitable for delivery of flow therapies, in which the RPT device generates the flow of air at controlled flow rates rather than controlled pressures. The "vent" at the unsealed patient interface 3800, through which excess airflow escapes to ambient, is the passage between the end of the prongs 3810a and 3810b of the cannula 3800 via the patient's nares to atmosphere.

If a patient interface is unable to comfortably deliver a minimum level of positive pressure to the airways, the patient interface may be unsuitable for respiratory pressure therapy.

The patient interface 3000 in accordance with one form of the present technology is constructed and arranged to be able to provide a supply of air at a positive pressure of at least 6 cmH₂O with respect to ambient.

The patient interface 3000 in accordance with one form of the present technology is constructed and arranged to be able to provide a supply of air at a positive pressure of at least 10 cmH₂O with respect to ambient.

The patient interface 3000 in accordance with one form of the present technology is constructed and arranged to be able to provide a supply of air at a positive pressure of at least 20 cmH₂O with respect to ambient.

### 5.3.1 Seal-forming structure

In one form of the present technology, a seal-forming structure 3100 provides a target seal-forming region, and may additionally provide a cushioning function. The target seal-forming region is a region on the seal-forming structure 3100 where sealing may occur. The region where sealing actually occurs- the actual sealing surface- may change within a given treatment session, from day to day, and from patient to patient, depending on a range of factors including for example, where the patient interface was placed on the face, tension in the positioning and stabilising structure and the shape of a patient's face.

In one form the target seal-forming region is located on an outside surface of the seal-forming structure 3100.

In certain forms of the present technology, the seal-forming structure 3100 is constructed from a biocompatible material, e.g. silicone rubber.

A seal-forming structure 3100 in accordance with the present technology may be constructed from a soft, flexible, resilient material such as silicone.

In certain forms of the present technology, a system is provided comprising more than one a seal-forming structure 3100, each being configured to correspond to a different size and/or shape range. For example the system may comprise one form of a seal-forming structure 3100 suitable for a large sized head, but not a small sized head and another suitable for a small sized head, but not a large sized head.

### 5.3.2 Plenum chamber

The plenum chamber 3200 has a perimeter that is shaped to be complementary to the surface contour of the face of an average person in the region where a seal will form in use. In use, a marginal edge of the plenum chamber 3200 is positioned in close proximity to an adjacent surface of the face. Actual contact with the face is provided by the seal-forming structure 3100. The seal-forming structure 3100 may extend in use about the entire perimeter of the plenum chamber 3200. In some forms, the plenum chamber 3200 and the seal-forming structure 3100 are formed from a single homogeneous piece of material.

In certain forms of the present technology, the plenum chamber 3200 does not cover the eyes of the patient in use. In other words, the eyes are outside the pressurised volume defined by the plenum chamber. Such forms tend to be less obtrusive and / or more comfortable for the wearer, which can improve compliance with therapy.

In certain forms of the present technology, the plenum chamber 3200 is constructed from a transparent material, e.g. a transparent polycarbonate. The use of a transparent material can reduce the obtrusiveness of the patient interface, and help improve compliance with therapy. The use of a transparent material can aid a clinician to observe how the patient interface is located and functioning.

In certain forms of the present technology, the plenum chamber 3200 is constructed from a translucent material. The use of a translucent material can reduce the obtrusiveness of the patient interface, and help improve compliance with therapy.

### 5.3.3 Positioning and stabilising structure

The seal-forming structure 3100 of the patient interface 3000 of the present technology may be held in sealing position in use by the positioning and stabilising structure 3300.

In one form the positioning and stabilising structure 3300 provides a retention force at least sufficient to overcome the effect of the positive pressure in the plenum chamber 3200 to lift off the face.

In one form the positioning and stabilising structure 3300 provides a retention force to overcome the effect of the gravitational force on the patient interface 3000.

In one form the positioning and stabilising structure 3300 provides a retention force as a safety margin to overcome the potential effect of disrupting forces on the patient interface 3000, such as from tube drag, or accidental interference with the patient interface.

In one form of the present technology, a positioning and stabilising structure 3300 is provided that is configured in a manner consistent with being worn by a patient while sleeping. In one example the positioning and stabilising structure 3300 has a low profile, or cross-sectional thickness, to reduce the perceived or actual bulk of the apparatus. In one example, the positioning and stabilising structure 3300 comprises at least one strap having a rectangular cross-section. In one example the positioning and stabilising structure 3300 comprises at least one flat strap.

### 5.3.4 Vent

In one form, the patient interface 3000 includes a vent 3400 constructed and arranged to allow for the washout of exhaled gases, e.g. carbon dioxide.

In certain forms the vent 3400 is configured to allow a continuous vent flow from an interior of the plenum chamber 3200 to ambient whilst the pressure within the plenum chamber is positive with respect to ambient. The vent 3400 is configured such that the vent flow rate has a magnitude sufficient to reduce rebreathing of exhaled CO₂ by the patient while maintaining the therapeutic pressure in the plenum chamber in use.

One form of vent 3400 in accordance with the present technology comprises a plurality of holes, for example, about 20 to about 80 holes, or about 40 to about 60 holes, or about 45 to about 55 holes.

The vent 3400 may be located in the plenum chamber 3200. Alternatively, the vent 3400 is located in a decoupling structure, e.g., a swivel.

In certain forms the vent is located in a part of the respiratory system other than the patient interface, for example the air circuit, between the air circuit and the patient interface or the positioning and stabilising structure, as described below.

Vents according to certain forms of the technology are described in more detail below.

### 5.3.5 Decoupling structure(s)

In one form the patient interface 3000 includes at least one decoupling structure, for example, a swivel or a ball and socket.

### 5.3.6 Connection port

Connection port 3600 allows for connection to the air circuit 4170.

### 5.3.7 Forehead support

In one form, the patient interface 3000 includes a forehead support 3700.

### 5.3.8 Anti-asphyxia valve

In one form, the patient interface 3000 includes an anti-asphyxia valve.

### 5.3.9 Ports

In one form of the present technology, a patient interface 3000 includes one or more ports that allow access to the volume within the plenum chamber 3200. In one form this allows a clinician to supply supplementary oxygen. In one form, this allows for the direct measurement of a property of gases within the plenum chamber 3200, such as the pressure.

### 5.4 RPT DEVICE

An RPT device 4000 in accordance with one aspect of the present technology comprises mechanical, pneumatic, and/or electrical components and is configured to execute one or more algorithms 4300, such as any of the methods, in whole or in part, described herein. The RPT device 4000 may be configured to generate a flow of air for delivery to a patient's airways, such as to treat one or more of the respiratory conditions described elsewhere in the present document.

### 5.5 AIR CIRCUIT

An air circuit 4170 in accordance with an aspect of the present technology is a conduit or a tube constructed and arranged to allow, in use, a flow of air to travel between two components such as RPT device 4000 and the patient interface 3000 or 3800.

### 5.6 VENT

### 5.6.1 Vent overview

Certain forms of respiratory therapy system may comprise a vent 3400 to allow for the washout of exhaled gases, e.g. carbon dioxide. Venting of gas from the respiratory therapy system may result in noise caused by the flow of air through the vent 3400, which noise can disturb the patient 1000 and/or the bed partner 1100.

The level and quality of noise produced by venting of gases may be different during inhalation compared to exhalation, with the noise usually being louder during exhalation because exhaled air flows in the opposite direction to, and impedes, air received from the RPT device 4000, which creates turbulence and therefore noise. The cyclical nature of the noise may be particularly undesirable.

Furthermore, the force of the air flow exiting the vent may disrupt, or be uncomfortable for, the patient 1000 and/or bed partner 1100. Air exiting the vent directly into the surrounding ambient air may also result in flow separation, particularly at high velocities. Flow separation causes an increase in turbulence in the flow of air and therefore noise. The faster the flow of air exiting the vent, the louder the noise associated with the vent 3400. Slowing down the flow of air in the vent may therefore reduce the noise associated with the vent 3400.

In certain forms of the present technology a vent 3400 may be provided in the form of a vent structure 6000 provided to, or comprised as part of, a part of the respiratory system. Vent 3400 may be configured to reduce the noise produced by venting gases and/or to reduce the speed of the air flow as it exits the vent.

In some forms, the vent structure 6000 may be formed from a plastics material, for example polycarbonate, including wholly formed from the plastics material. In some forms the vent structure 6000 is formed from a combination of different materials.

In certain forms, the vent structure 6000 may have the advantage of not comprising any diffuser material as is present in some prior vents to reduce jetting of the air flow. Diffuser material is an additional component to a patient interface so adds cost and complexity in manufacture, assembly and use, and may need to be cleaned and/or replaced regularly.

In certain forms of the present technology, the respiratory system may comprise a further vent structure 6000 in addition to the vent 3400 for venting exhaled gases. The further vent structure 6000 may be used for reducing the flow rate of air delivered to the patient 1000 from the RPT device 4000. The further vent structure 6000 may be in the form of, or comprised as part of, a connection member 7000, as described below, and may be located in-line with the air circuit 4170.

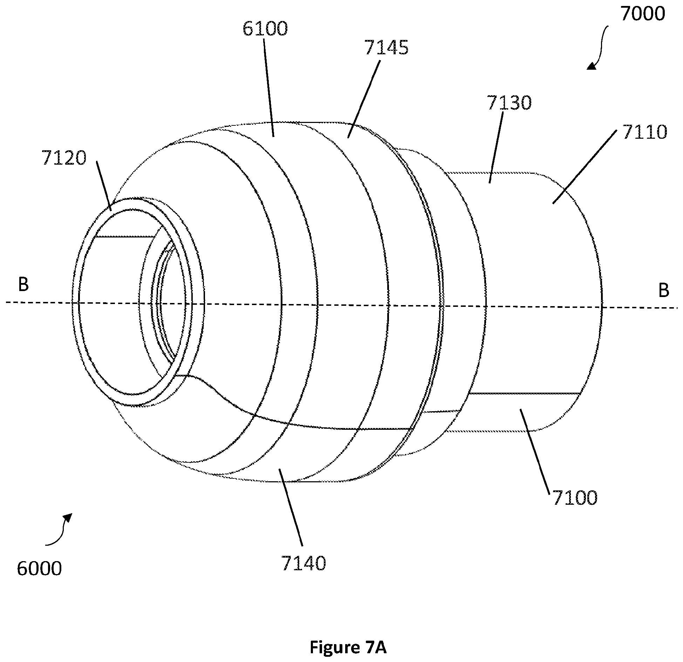

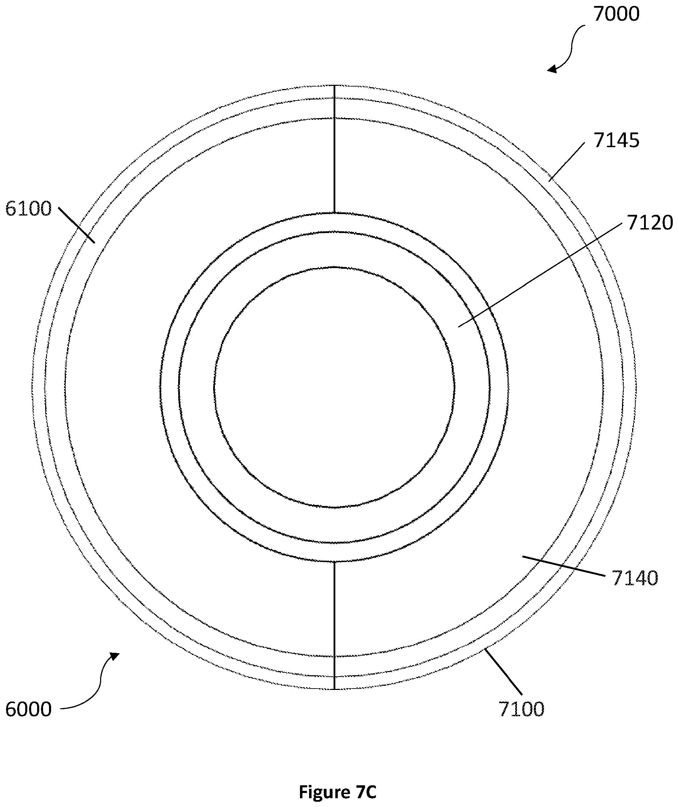

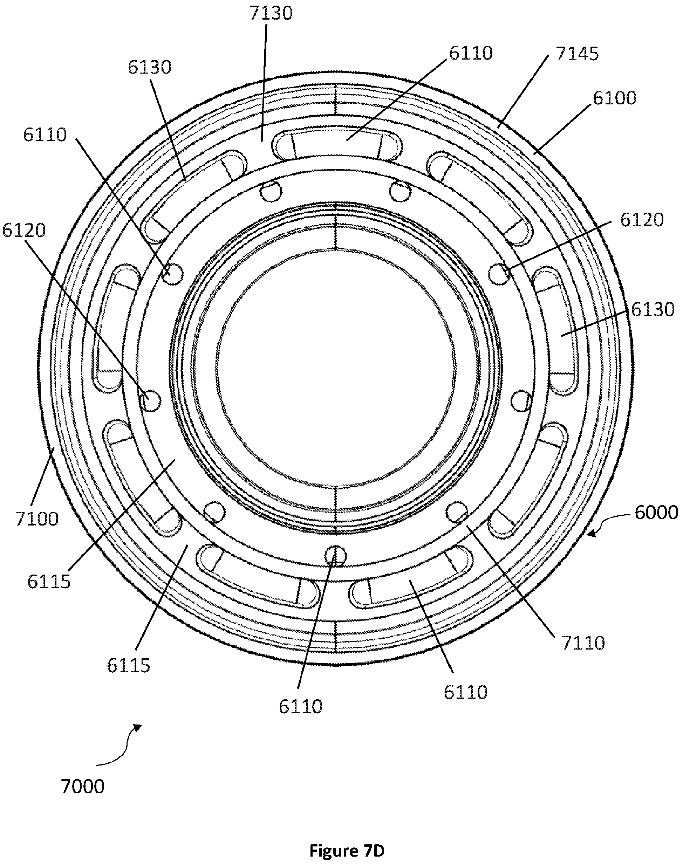

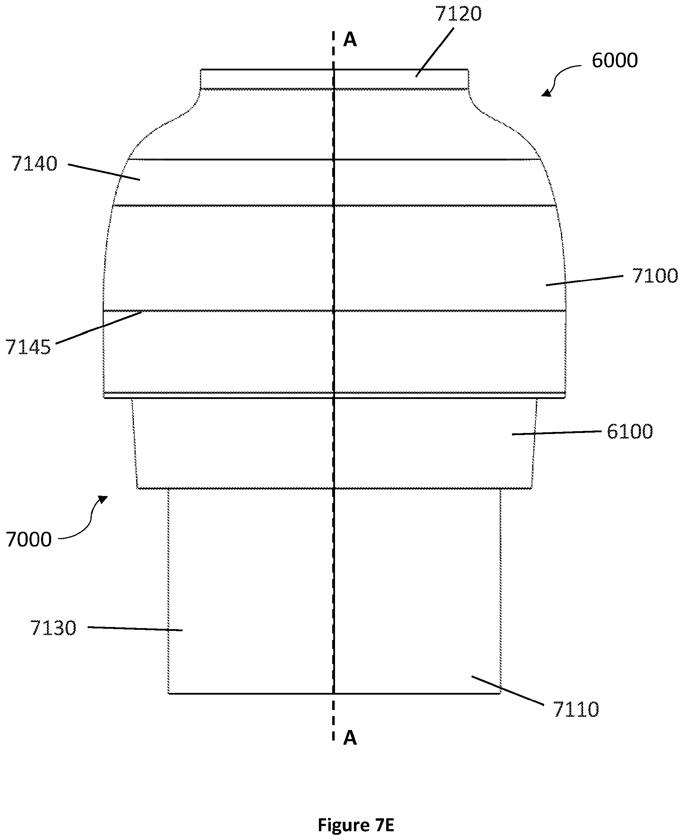

In the forms of the technology shown in Figures 7A to 7F the vent structure has been designed to achieve a significant amount of reduction in the noise of the air flow through the vent and the force of the air flow exiting the vent for the following conditions: a flow rate exiting the vent of between approximately 11-12 L/min; a mask pressure of 10cm H₂O; and a Reynolds number of approximately 1000. The design, and in particular various aspects of the geometry, of the vent structure may change when designed for different conditions. The flow rate of air exiting the vent is determined as approximately one-third of 36 L/min which is the total flow rate that is released from the respiratory system into the surrounding ambient air. This value was determined as a balance between keeping the flow of air through the vent relatively low in order to avoid significant noise and the requirement to vent air from the mask to prevent CO₂ build-up in the mask and re-breathing by the patient.

### 5.6.2 Location of the vent structure

### 5.6.2.1 Connection member

In certain forms of the present technology the vent structure 6000 may be formed as part of a connection member 7000. Figures 7A to 7F illustrate a connection member 7000 according to certain forms of the technology. The connection member 7000 is configured to form part of the respiratory therapy system connected between the air circuit 4170 and the patient interface 3000. In some forms of the technology this may be an indirect connection, for instance there may be one or more additional components that connect between the connection member 7000 and the air circuit 4170 or between the connection member 7000 and the patient interface 3000. In other forms the connection member 7000 is directly connected to the air circuit 4170 and/or is directly connected to the patient interface 3000. In forms of the technology, the connection member 7000 fluidly connects the air circuit 4170 to the patient interface 3000, i.e. it allows the flow of air from the air circuit 4170 to the patient interface 3000.

In some forms of the technology the connection member 7000 may be a separate component to, and may be separable from, the air circuit 4170 and/or the patient interface 3000. In other forms of the technology the air circuit 4170 or the patient interface 3000 comprises the connection member 7000.

In one form the connection member 7000 may comprise a tube portion 7100 configured to directly or indirectly fluidly connect the air circuit 4170 to the patient interface 3000. The tube portion 7100 may be a hollow cylinder (i.e. with a circular cross-section) or assembly of components forming a generally cylindrical shape with an air path through it to convey air directly or indirectly from the air circuit 4170 to the patient interface 3000. In other forms of the technology the tube portion may have a different cross-sectional shape, for example oval, D-shaped or polygonal. The tube portion 7100 may comprise a first end 7110 configured to directly or indirectly fluidly connect to the air circuit 4170 and a second end 7120 configured to directly or indirectly fluidly connect to the patient interface 3000.

The connection member 7000 may further comprise a vent structure 6000 configured to allow air in the tube portion 7100 to exit into the surrounding ambient air. Such a vent structure 6000 is described in further detail below in relation to Figures 7A-7F.

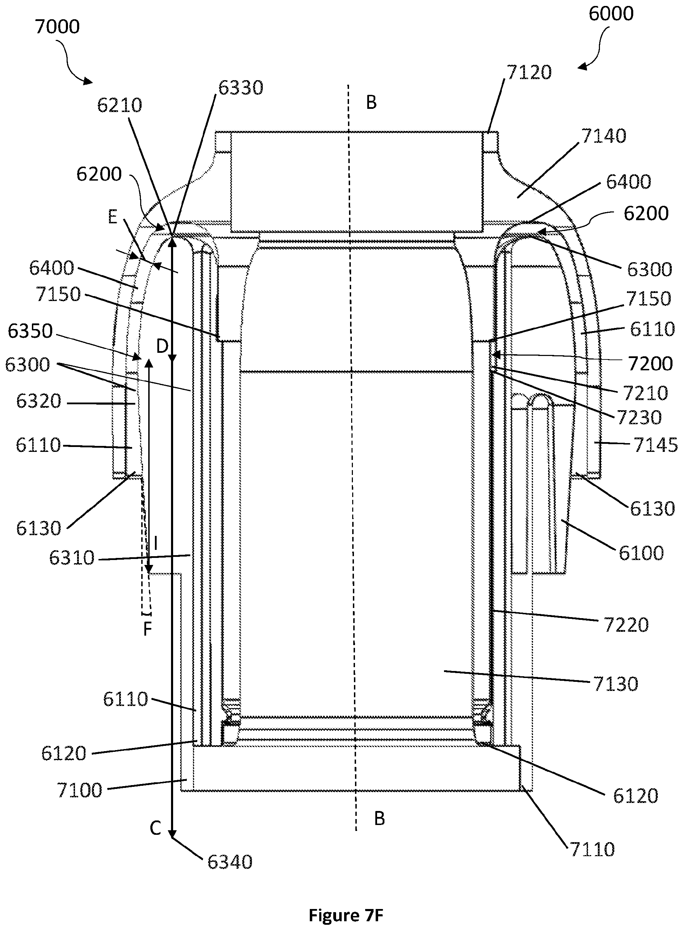

In one form of the technology the tube portion 7100 comprises an outer tube portion 7130 and an inner tube portion 7140. The outer tube portion 7130 may be rotatable relative to the inner tube portion 7140 around a mutual longitudinal axis B. At least a portion of the inner tube portion 7140 is positioned inside at least a portion of the outer tube portion 7130. As shown in Figure 7F, an end stop 7150 may set the position of the inner tube portion 7140 and the outer tube portion 7130 relative to one another. The end stop 7150 may be in the form of flanges on either or both the outer tube portion 7130 and the inner tube portion 7140. Other forms of end stop 7150 may be provided in other forms of the technology.

In one form, the outer tube portion 7130 may comprise the first end 7110, i.e. the outer tube portion 7130 may be configured to connect to the air circuit 4170, and the inner tube portion 7140 may comprise the second end 7120, i.e. the inner tube portion 7140 may be configured to connect to the patient interface 3000. In other forms the outer tube portion 7130 may comprise the second end 7120 and the inner tube portion 7140 comprises the first end 7110. The connections of the first end 7110 and/or the second end 7120 to the air circuit 4170 and/or the patient interface 3000, or to components intermediary between them, for example other lengths of conduit, may be achieved via a decoupling arrangement including a ball and socket or a swivel ring arrangement. Alternatively, the connections may be removably connected by a clip arrangement, a screw and thread arrangement, or a snap-fit arrangement. In some forms of the technology the first end 7110 and/or the second end 7120 may be integrally formed with another component for instance a portion of the air circuit 4170 or the patient interface 3000.

Avoiding locating the vent 3400 on the patient interface 3000, for example between the air circuit 4170 and the patient interface 3000, increases the distance between the patient 1000 and the vent 3400. This may help to reduce the noise of venting gases as perceived by the patient.

### 5.6.2.2 Patient Interface

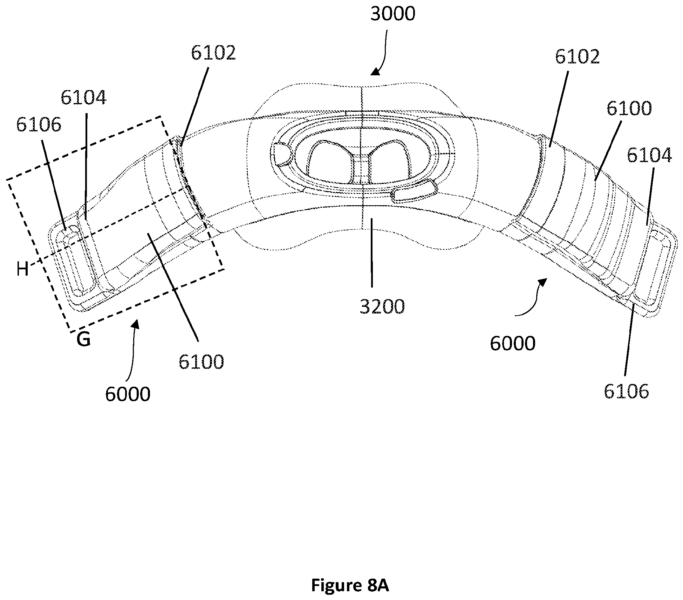

In some forms of the technology, a patient interface 3000 comprising a plenum chamber 3200 and a seal-forming structure 3100 as described above may further comprise a vent structure 6000, as described below in relation to Figures 8A to 8D. The vent structure 6000 allows a continuous flow of gases exhaled by the patient 1000 from an interior of the plenum chamber to be vented to ambient. The vent structure 6000 is preferably sized and shaped to maintain the therapeutic pressure in the plenum chamber 3200 in use. In these forms, the vent structure 6000 may be structured and arranged in a form that is specifically adapted to the geometry of the patient interface 3000.

In some forms of the technology the vent structure 6000 is provided to a portion of the plenum chamber 3200, for example a portion that is on an anterior side of the plenum chamber 3200 when the patient interface 3000 is donned by a patient 1000.

### 5.6.3 Vent structure

### 5.6.3.1 Vent housing

In certain forms of the technology, the vent structure 6000 comprises a vent housing 6100. The vent housing 6100 may comprise one or more components that collectively define one or more flow paths 6110 for a flow of air being vented from the respiratory system. The vent housing 6100 may further define a vent inlet 6120 configured to allow the flow of air being vented to enter the flow path(s) 6110 and a vent outlet 6130 configured to allow the flow of air being vented to exit the flow path into the surrounding ambient air. The flow path(s) 6110 fluidly connect(s) the vent inlet 6120 to the vent outlet 6130. The flow path(s) 6110 generally have a length substantially greater than the width or height of the flow path(s).

It will be appreciated that, in forms of the technology, the flow paths 6110 are spaces formed within, or partially within, the vent housing 6100. Therefore, the shape and configuration of the flow paths 6110 are determined by the shape and configuration of the vent housing 6100, especially those portions of the vent housing 6100 that contact the flow paths 6110. In this specification, where reference is made to the shape and/or configuration of a flow path 6110, it should be appreciated that this shape / configuration is provided by virtue of the shape / configuration of the vent housing 6100 defining the flow path 6110.

In some forms of the technology the vent housing 6100 comprises a plurality of partitions 6115 which form a plurality of flow paths 6110 therebetween. The flow paths 6110 may all be of a similar form. For example, the connection member 7000 shown in Figures 7A to 7D has cylindrical symmetry and each of the flow paths 6110 is symmetrically similar around a circumference of the cylindrical form. In another example, the flow paths 6110 within the patient interfaces 3000 shown in Figures 8A to 8D are similar to each other and arranged in parallel to each other. For the purposes of the ensuing description, where a single flow path 6110 will be described in detail it should be understood that this description may also apply to the other flow paths 6110, if present.

In forms of the technology where the vent structure 6000 forms part of the connection member 7000, as described above, the partitions may be formed as part of the inner tube portion 7140 and/or the outer tube portion 7130.

The vent housing 6100 may comprise one or more features, such as described in later sections, that assist in reducing the noise of the flow of air through the vent structure 6000.

### 5.6.3.1.1 Connection member

It has already been explained that in some forms of the technology the vent structure 6000 may be comprised as part of a connection member 7000, for example as shown in Figures 7A to 7F. Consequently, the vent housing 6100 may be comprised as part of connection member 7000 in some forms.

In such forms, the vent housing 6100 may be formed from, or comprised as part of, a tube portion 7100. In some forms, the vent housing 6100 may be formed from, or comprised as part of, an outer tube portion 7130 and an inner tube portion 7140 where the outer and inner tube portions are arranged co-axially with the outer tube portion 7130 generally radially outside the inner tube portion 7140. That is, these tube components contribute to defining the flow path(s) 6110 of the vent 3400. One advantage of the vent housing 6100 comprising both an outer tube portion 7130 and an inner tube portion 7140 is that the two tube portions can be arranged rotatable relative to each other so that components attached to each end (for example different parts of an air circuit 4170) can rotate longitudinally relative to each other.

In such forms, the vent inlet 6120 may be positioned closer to the first end 7110 of the connection member 7000 than the vent outlet, and the vent outlet 6130 may be positioned closer to the second end 7120 of the connection member 7000 than the vent inlet. In other forms, the vent inlet 6120 may be positioned closer to the second end 7120 and the vent outlet 6130 may be positioned closer to the first end 7110.

In the form shown in Figures 7A to 7F, the vent housing 6100 is configured such that each flow path 6110 comprises at least a part that is oriented substantially parallel to a longitudinal axis B of the connection member 7000. In forms where the connection member 7000 comprises an outer tube portion 7130 and an inner tube portion 7140 each flow path 6110 may have a part arranged substantially parallel to the longitudinal axis of both tube portions, i.e. the mutual longitudinal axis B. In some forms the flow path 6110 may be formed entirely within the vent housing 6100, e.g. entirely within the outer tube portion 7130, entirely within the inner tube portion 7140, or between the outer tube portion 7130 and the inner tube portion 7140. As will be discussed below, each flow path 6110 may additionally comprise at least a part that is oriented at a non-parallel angle to the longitudinal axis of the connection member 7000.

Other features of the vent housing 6100 in forms of the technology in which the vent housing 6100 is comprised as part of connection member 7000 will be described below with reference to features of the vent structure 6000 in certain forms of the technology.

### 5.6.3.1.2 Patient interface

In other forms of the technology, for example as shown in Figures 8A to 8D, the vent housing 6100 may be comprised as part of a patient interface 3000. In such forms, the vent housing 6100 may be comprised as part of, or may be provided to, the plenum chamber 3200. In the examples of Figures 8A to 8D, there are two vent housings 6100 provided on either lateral side of the patient interface 3000.

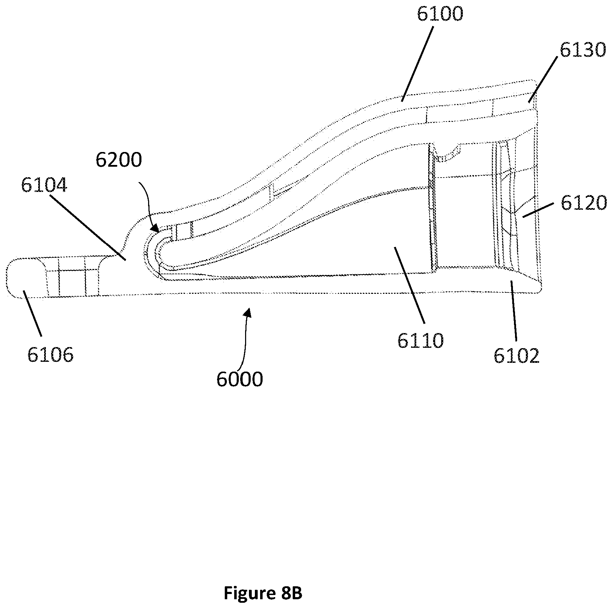

In the example of Figures 8A and 8B, each vent housing 6100 is a separate component to the component (e.g. frame) forming the planum chamber 3200. Each vent housing 6100 has a first end 6102 and a second end 6104. The first end 6102 is configured to fluidly connect to an opening on a lateral side of the plenum chamber 3200. In the form shown, the first end 6102 comprises an opening that mounts to a tube on a lateral side of the plenum chamber 3200, for example by a friction-fit or snap-fit connection. The second end 6104 extends away from the plenum chamber 3200 and comprises a connector 6106 configured to, in use, connect to the positioning and stabilising structure 3300. For example, the connector 6106 may take the form of a loop or clip.

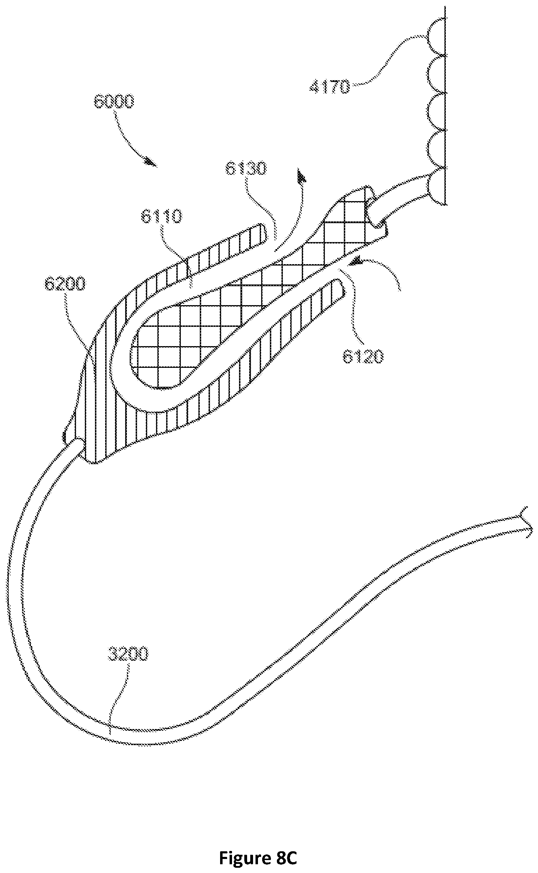

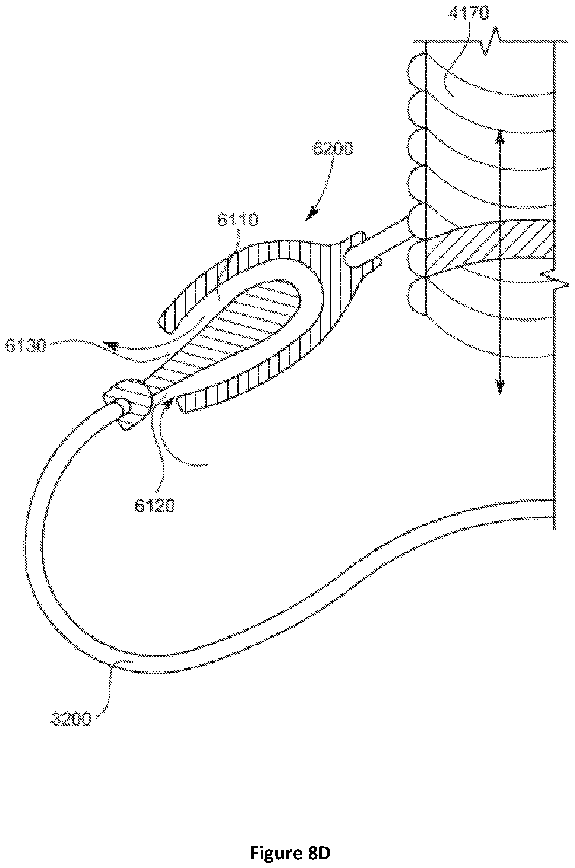

In the examples of Figures 8C and 8D, the vent housing 6100 is provided to the surface of the plenum chamber 3200. The vent housing 6100 and the flow path(s) 6110 may be configured to follow the general shape, i.e. curvature, of the plenum chamber 3200. The vent housing 6100 may be formed by one or more walls of the plenum chamber 3200 or may be formed by an insert provided to one or more walls of the plenum chamber 3200. In the forms of the technology shown in Figures 8C and 8D the vent structure 6000 is provided to an anterior wall of the plenum chamber 3200 in lateral regions. In another form, the vent structure 6000 may be provided to a medial region of the plenum chamber 3200.

### 5.6.3.2 Shape of flow paths

In certain forms of the technology, the vent housing 6100 is configured so that each flow path 6110 comprises a curved turning region 6200 in which the flow path 6110 changes direction by at least 90°. The turning region 6200 generally refers to the region of the flow path in which the change of direction occurs. The turning region 6200 is curved so that air flowing through the region is caused to change direction smoothly. For example, in some forms the turning region does not include any angles or abrupt changes in direction. This may be described mathematically as the gradient of the walls of the curved turning region being a continuous function.

A flow path 6110 that includes a turning region 6200 which changes the direction of the flow of air through the vent may provide several advantages. The flow of air along a flow path reduces the velocity of the flow of air due to friction of the air with the walls of the flow path. Therefore, generally speaking, the longer the flow path, the greater the surface area of the flow path that contacts the flow of air, and the more the velocity of the air flow is reduced through friction. However, there are limits to the length of flow path that can be practically achieved in a component within a respiratory therapy system without being detrimental to the compactness and ease-of-use of the component. Changing the direction of the flow path 6110 allows the flow path 6110 to be longer that it otherwise would be without significantly increasing the length of the component that comprises the vent housing 6100.

The amount of noise that is produced when venting air from a vent structure in a respiratory therapy system is related to the velocity of the vented air. Generally, more noise is created when air is vented at a higher velocity. Therefore, reducing the velocity of the vented air reduces the amount of noise created by the vent.

The ability to increase the length of the flow path 6110 without also increasing the length of the vent housing 6100 allows for a relatively compact vent design while also reducing the noise produced by the vent. A compact vent design may be particularly beneficial in circumstances where the vent structure 6000 forms part of a rigid component, in which case a rigid component with a shorter length will reduce the overall rigidity of the mask system. A compact vent design will also reduce the weight of the vent and may therefore improve the comfort for the patient.

In certain forms of the technology, the angle of the turn in the turning region 6200 may be anywhere between substantially 90° and a turn that causes the flow path to change direction to a substantially opposite direction. In some forms of the technology, the longitudinal cross-section of the flow path 6110 is substantially U-shaped due to the turning region 6200. According to the invention, the angle of the turn is substantially 180°. In general, the larger the angle of the turn, the more the velocity of the flow of air is reduced and the more kinetic energy is dissipated leading to less noise being produced. A suitable angle of the turning region 6200 may be dependent on the nature of the flow of air through the flow paths 6110. For example, a change in direction of a smaller angle may be sufficient if the flow rate or velocity of air entering the flow path 6110 is low.

In the form of the technology shown in Figures 7A to 7F the vent structure is comprised as part of a connection member 7000. In the exemplary illustrated form, each flow path is configured so that air enters the flow path through a vent inlet 6120 proximate the first end 7110 of the connection member 7000 and flows through a part of the flow path 6110 upstream of the turning region 6200 in a direction parallel to the longitudinal axis B of the connection member 7000. In the form of the technology shown in Figure 7F, this part of each flow path 6110 is formed as a hole through the outer tube portion 7130.

There is a change in direction of the flow path 6110 in the turning region 6200 which causes air flowing through the flow path to be diverted away from the central axis B of the tube portion 7100. According to the invention, the turning region 6200 is configured to continue diverting the flow of air and, as shown in Figure 7F, the flow path 6110 changes direction by an angle of substantially 180° before venting the air through the vent outlet 6130. Therefore, the flow of air exiting the vent outlet 6130 is flowing in a direction that is substantially parallel to the outer surface of the outer tube portion 7130 and parallel to the longitudinal axis B of the connection member 7000. In some forms, the turning region 6200 is configured so that the direction of air vented from the vent outlet 6130 is at an angle that is greater than 180° from the angle of the air through the vent inlet 6120, i.e. so that air exiting the vent outlet 6130 flows towards an outer surface of the outer tube portion 7130. Venting the flow of air exiting the vent structure 6000 in a direction that is parallel to the outer wall of the connection member 7000, or directed slightly towards the outer wall of the connection member 7000 may avoid venting air towards the patient 1000 or bed partner 1100 and causing discomfort. The flow of air exiting the vent structure 6000 may also be less turbulent due to the Coanda effect which results in the flow of air remaining close to the tube portion 7100 after leaving the vent.

In the form of the technology shown in Figure 7F, the part of each flow path 6110 that is downstream of the turning region 6200 is formed between an outer surface of the outer tube portion 7130 and an inner surface of a flange 7145 comprised as part of the inner tube portion 7140. The flange 7145, although comprised as part of the inner tube portion 7140 extends radially outside the outer tube portion 7130 from the perspective of the cylindrical geometry of the connection member 7000. In the illustrated form, flange 7145 extends radially outwardly from the main body of the inner tube portion 7140 proximate the second end 7120 of the tube portion 7100 and curves downwardly towards the first end 7110 of the tube portion 7100 so that the distal end of the flange 7145 (and much of the body of the flange) is positioned radially outside the outer tube portion 7130. This configuration means that an inner path surface 6300 on the inner side of the curved flow path 6110 is a surface of the outer tube portion 7130 and an outer path surface 6400 on an outer side of the curved flow path 6110 is a surface of the inner tube portion 7140.

In the forms of the technology illustrated in Figures 8A to 8C, the vent structure 6000 is comprised as part of, or is provided to, the plenum chamber 3200. The flow path 6110 comprises a vent inlet 6120 on an inside surface of the plenum chamber 3200 that is oriented substantially parallel to an anterior wall of the plenum chamber 3200. In the illustrated form, air flows along the flow path 6110 after entering through the vent inlet 6120 in a lateral direction, i.e. away from a centre region of the patient interface 3000 (for example a region comprising connection port 3600). The curved turning region 6200 causes air flowing through the flow path 6110 to be turned back in a medial direction so that the air exits through the vent outlet 6130 flowing towards a centre region of the patient interface, e.g. towards a conduit connected to a connection port 3600 in a central anterior region of the patient interface 3000.

In the form of the technology illustrated in Figure 8D, the vent structure 6000 is located in an anterior wall of the plenum chamber 3200 lateral to the central connection port 3600 (and a similar vent structure 6000 may be located on the other side of the patient interface 3000, but is not illustrated), similar to the vent structure 6000 in Figure 8C, but is oriented the other way around. That is, air flows into the vent inlet 6120 in a medial direction and air exits the vent outlet 6130 in a lateral direction.

### 5.6.3.2.1 Cross-sectional shape of turning region

In forms of the technology where the vent housing comprises a curved turning region 6200, the vent housing 6100 comprises an inner path surface 6300 on an inner side of the curved flow path 6110 and an outer path surface 6400 on an outer side of the curved flow path 6110. In some forms of the technology, in the turning region 6200, the inner path surface 6300 has a cross-sectional shape in the form of a segment of a circle or an arc. In some forms, only a portion of the inner path surface 6300 in the turning region 6200 may have a cross-sectional shape in this form, i.e. there may also be a portion of the inner path surface 6300 in the turning region 6200 that takes another form, for example a curved segment of another shape.

For example, in the form of the technology shown in Figures 7F, 8B, 8C and 8D, the portion 6210 of the turning region has the cross-sectional shape in the form of a segment of a circle.

This shape may assist in preventing the flow of air through the turning region 6200 from separating from, or detaching from, the inner surface 6400 of the flow path 6110. Such flow separation / detachment may add turbulence to the flow of air through the flow path, which may result in additional noise being generated by the vent in use.

### 5.6.3.2.2 Cross-sectional shape of vent structure

In some forms of the technology where the vent housing comprises a curved turning region 6200, the inner path surface 6300 of the vent housing 6100 has a cross-sectional shape in, or resembling, the form of part of an aerofoil. An aerofoil, or airfoil, is a cross-sectional shape with curved surfaces that is typically used in wings, fins, and tailplanes of aircraft to create lift when moving through a fluid. One typical property of an aerofoil is that air flows smoothly across its surface without creating turbulence or air separation. By configuring inner path surface 6300 with a cross-sectional shape in the shape of an aerofoil, this property creates beneficial effects for a vent structure in a respiratory therapy system. For example, the aerofoil shape (specifically the relatively high length-to-drag ratio of such a shape compared to other shapes) creates a low level of detachment of air from the surfaces or walls forming the flow path 6110 as air flows through it. This reduces the amount of turbulence created in the flow of air, reducing the noise produced by the flow of air through the flow path 6110.

It will be appreciated that there are various different cross-sectional shapes that may be considered to be aerofoils. In some forms, the cross-sectional shape of the inner path surface 6300 may not have the exact shape of any specific aerofoil but may have a curved shape resembling an aerofoil which provides the advantage of generating low levels of turbulence, as described above. The shape of the inner path surface 6300 may be modified from some aerofoil shapes, for instance to have a shape that is easier to tool and manufacture.

Furthermore, an aerofoil shape forms a closed loop, i.e. a surface on one side of the aerofoil meets the surface on the other side at a leading edge and a trailing edge. In forms of the technology the inner path surface 6300 does not form such a closed loop. The inner path surface 6300 may comprise a point equivalent to the leading edge of an aerofoil, which forms part of the curved turning region 6200. In some forms, the inner path surface 6300 does not comprise a point equivalent to the trailing edge of an aerofoil. Nevertheless, the shape of the part of the inner path surface 6300 that resembles an aerofoil will be described with reference to the terminology of an aerofoil as if the full aerofoil profile was present.

By adjusting the cross-sectional shape of the vent structure 6000, for example by adjusting the aerofoil shape, the point along the flow path 6110 at which the flow of air transitions from turbulent to laminar may be varied. In some forms it is desirable to configure the cross-sectional shape so that this transition point is as far from the vent outlet 6130 as possible. This assists in reducing the amount of noise generated inside the flow path 6110 that is propagated to the vent outlet 6130, and consequently particularly audible. Experimentation with different cross-sectional shapes can be used to identify suitable cross-sectional shapes taking this consideration into account.

In the form of the technology shown in Figure 7F, the cross-sectional shape of the inner path surface 6300 comprises a longitudinally flat region 6310 upstream of the turning region 6200. Downstream of the turning region 6200, the inner path surface 6300 comprises a curved region 6320 with a smooth curvature having no indentations. As shown in Figure 7F, the curved region 6320 continues after the vent outlet 6130 but does not continue all the way to meet with the longitudinally flat region 6310 to form a trailing edge as with a typical aerofoil shape. The inner path surface 6300 therefore does not form an entire aerofoil shape but a portion of an aerofoil shape. The inner path surface 6300 in the forms of the technology shown in Figures 8C and 8D may be considered to be closer to a 'full' aerofoil-like shape since the vent housing 6100 is relatively thin in a region distal from the leading edge in the curved turning region 6200.

As shown in Figure 7F, the part-aerofoil shape of the inner path surface 6300 defines a chord line C, i.e. the straight-line distance between the trailing edge and the leading edge of the aerofoil shape, if the aerofoil-shaped part of the surface was extended to form a closed loop. The chord line may have a chord length of at least 50mm, for example the chord length may be 54mm. A small chord length may be desirable in order to provide a compact vent design for the reasons discussed above. An alternative way of describing the chord length in certain forms of the technology is as a length defined by a straight-line distance starting at a first point 6330 on the inner path surface 6300 that is furthest from the vent inlet 6120 to a second point 6340 that is located furthest from the first point 6330 if the curved region 6320 extended along its trajectory to meet the straight line. In the forms of technology where the vent structure 6000 is in the form of a connection member 7000, such as is shown in Figure 7F, the chord length may be substantially parallel to the longitudinal axis B of the tube portion.

An aerofoil-like shape of an inner path surface 6300 according to certain forms of the technology has a region of maximum thickness 6350. This region is shown in the form of the technology shown in Figure 7F as being located along the chord line C at a distance D from the leading edge of the aerofoil. In certain forms of the technology, distance D is between 25% and 40% of the chord length. For example, in forms of the technology in which the chord length is approximately 50-54mm, the distance D along the chord line to the region of maximum thickness 6350 is in the range of 12.5mm-22mm, for example in some forms of the technology this distance is 13.5mm. This has been found to be effective in keeping the amount of turbulence created by the flow path to be low, and to achieve a satisfactory balance between diffusivity and noise.

An alternative description of the region of maximum thickness 6350 is the region on the inner path surface 6300 where the perpendicular distance between the longitudinally flat region 6310 and the curved region 6320 is the greatest.

As shown in Figures 7F, 8C and 8D, in some forms of the technology, the vent outlet 6130 is located downstream of the region of maximum thickness 6350. The outer path surface 6400 therefore covers the inner path surface 6300 to at least the region of maximum thickness 6350 in these forms. This arrangement has been found to be effective at avoiding flow separation as flow exits the flow paths 6110, which can result in turbulence and increase the noise levels. In some forms of the technology, flow separation may occur at a point along the flow path 6110 adjacent and downstream of the region of maximum thickness 6350. The vent outlet 6130 may be located at a distance I past the point where flow separation occurs. Distance I may be a length between one and two times that of the width of the region of maximum thickness 6350. In some forms of the technology the distance I may be in the range of 6mm-12mm. The outer path surface 6400 therefore covers the inner path surface 6300 for a distance I past the region of maximum thickness 6350 in these forms.

### 5.6.3.3 Width of the flow path

In some forms of the technology, the width E of the flow path 6110 at the turning region 6200 and at a region downstream of the turning region 6200 is of a size that is sufficiently large to avoid water droplets in the flow of air blocking the flow path 6110 while being sufficiently small to regulate the flow rate. The width E is marked on the form of the technology shown in Figure 7F by way of example. The region downstream of the turning region has also been described as the curved region 6320 above. The region downstream of the turning region 6200 having the stated width may be the region between the turning region 6200 and the point on the flow path in line with the region of maximum thickness 6350 of the aerofoil-like shape. In some forms of the technology the width of the flow path 6110 may be in the range 0.75mm-1.5mm. It has been found that a width of the flow path 6110 of at least 0.75mm avoids water droplets in the flow of air blocking the flow path 6110. A maximum width of the flow path 6110 of 1.5mm may be used in order to regulate the flow rate. In certain forms, the width of the flow path 6110 may be at least 0.85mm since it has been found experimentally that this width is a particularly good balance between the competing considerations on the width of the flow path 6110 explained above.

In some forms of the technology, the width E of the flow path 6110 at the turning region 6200 may be the minimum width of the flow path 6110 along its length. Consequently, the width E of the flow path 6110 at the turning region 6200 may be described as the minimum distance of the gap between the inner path surface 6300 and the outer path surface 6400.

In the form of the technology shown in Figures 8A and 8B, the vent inlet 6120 is significantly wider than the width of each flow path 6110 for most of its length. The breadth of the vent inlet 6120 enables the inlet to receive air from the wide connectors 6016 on each side of the plenum chamber 3200, although it will be appreciated that, in other forms where the connectors have a different width, the vent inlet 6120 may also have a different width correspondingly. In the illustrated form, the vent inlet 6120 tapers to a narrower flow path 6110 in the turning region 6200, for example tapering to a width of approximately 0.85mm. Smooth inner walls of the vent housing 6100 promote low-turbulence flow through the flow path to keep noise levels low. In this form, each flow path 6110 downstream of the turning region 6200 curves around the tapering vent inlet 6120 so that air can be vented out of the vent outlet 6130 in approximately the opposite direction from which air flows into the vent inlet 6120.

### 5.6.3.4 Opening angle at the vent outlet

In some forms of the technology, the inner path surface 6300 of the vent housing 6100 at the vent outlet 6130 and the outer path surface 6400 of the vent housing 6100 at the vent outlet 6130 define an opening angle. In these forms the opening angle is the angle between these surfaces at the vent outlet 6130 when the flow path 6110 is viewed in cross-section along the length of the flow path 6110. In the form of the technology shown in Figure 7F, the opening angle is marked as angle F. In certain forms, the opening angle is sufficiently small to assist in reducing detachment and turbulence of the air exiting the vent outlet 6130 and therefore reducing noise generation to a desired level. In certain forms, the opening angle is substantially 10° or less. In certain forms, such as the form illustrated, the opening angle is substantially 7° or less. It has been found that an opening angle of 7° or less assists in reducing detachment and turbulence of the air exiting the vent outlet 6130 and therefore noise generation.

In the form of the technology shown in Figure 7F, if the outer path surface 6400 is oriented approximately parallel with the longitudinal axis B of the tube portion 7100 then a small opening angle between the inner path surface 6300 and the outer path surface 6400 at the vent outlet 6130 helps to direct the flow of air exiting the vent outlet 6130 towards the inner path surface 6300. As has been explained previously, this helps to reduce disturbance for the patient by maintaining the vent flow of air close to the air circuit 4170.

### 5.6.3.5 Vent housing parameters and flow regimes

As has been described, the vent housing 6100 may comprise one or more features that assist in reducing the noise of the flow of air through the vent structure 6000. Various parameters relating to those features have been mentioned by way of example. The features of the vent housing 6100 and the parameters to apply to those features may be selected in order to achieve the desired level of noise reduction for the types of flow regime that may be encountered with the apparatus. The flow regime may encompass, for example, the flow rate of air flowing through the respiratory therapy system (and consequently the vent structure 6000).

In other forms of the technology, including forms operating under different flow regimes, one or more features and/or parameters of the vent structure 6000 may be altered to provide a vent structure to achieve desired outcomes, for example a desired level of noise while achieving desired vent flow rates.

### 5.6.3.6 Noise attenuation structure

In some forms of the technology, for example as shown in Figures 7A-7F, the vent structure 6000 is comprised as part of a connection member 7000 as described above. The connection member may comprise a vent housing 6100, as described above. The vent housing 6100 may comprise an outer tube portion 7130 and an inner tube portion 7140 as previously described. In some forms, part of each of the flow paths 6110 may be formed by a part of the gap 7220 between the outer tube portion 7130 and the inner tube portion 7140. However, in some forms, the vent flow of air through this gap 7220 may be considered undesirable as it may be difficult to control the tolerances of the gap 7220 between the tube portions to create a flow path having the desired form. Consequently, in other forms, for example the form shown in Figures 7A to 7F, the outer tube portion 7130 may solely define a part of a flow path 6110, for example a part of the flow path 6110 upstream from the turning region 6200.

In this form, the end of the outer tube portion 7130 at the first end 7110 of the tube portion 7100 extends further than the end of the inner tube portion 7140 at the same end. The vent inlet 6120 is formed in an inner wall of a part of the outer tube portion 7130 that is level with, or extends further than, the end of the inner tube portion 7140 so that the vent inlet 6120 is exposed to the inside of the tube portion 7100 and can allow the flow of air to enter the flow path 6110 from inside the inner tube portion 7140.

In the form of the technology shown in Figures 7A to 7F, the part of the flow path 6110 solely defined by the outer tube portion 7130 is a first flow path portion and is formed in part by the longitudinally flat region 6310 of the inner path surface 6300 upstream of the turning region 6200 as described above. Consequently, the first flow path portion may be substantially parallel to the longitudinal axis B of the connection member 7000.

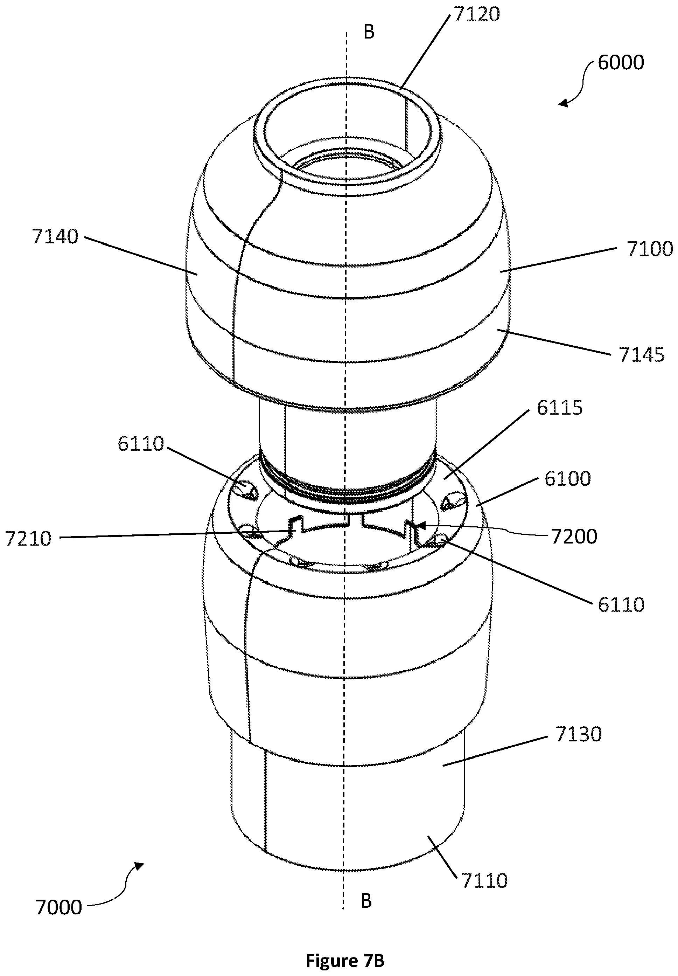

Each flow path 6110 may further comprise a second flow path portion defined between the inner tube portion 7140 and the outer tube portion 7130. The second flow path portion is downstream of the first flow path portion so that the second flow path portion receives the flow of air from the first flow path portion and delivers the flow of air to the vent outlet 6130. Openings at the end of the first flow path portions that allow vented gas to pass into the second flow path portions are shown in Figure 7B. These openings may be formed in an end region of the inner tube portion 7140 proximate the second end 7120 of the connection member. The second flow path portion may comprise the turning region 6200 and the parts of the flow path 6110 that are downstream of the turning region 6200, for example the curved region 6320 described above.

It is desirable for vented gases to flow through the flow path, i.e. through the vent inlet 6120, along the first flow path portion, along the second flow path portion and then out the vent outlet 6130. Nevertheless, there may be a gap 7220 between the outer tube portion 7130 and the inner tube portion 7140 (to enable the outer tube portion 7130 to rotate relative to the inner tube portion 7140 as explained above) and the gap 7220 may be fluidly connected to the flow path 6110. Consequently, the second flow path portion may also receive air from the gap 7220 between the outer tube portion 7130 and the inner tube portion 7140.

The vent structure 6000 may further comprise a noise attenuation structure 7200 for reducing noise generated by a flow of air through the gap 7220 between the outer tube portion 7130 and the inner tube portion 7140.

In one form, for example the form of the technology illustrated in Figure 7F, the noise attenuation structure 7200 comprises portions of the inner tube portion 7140 and/or the outer tube portion 7130 configured to form an acoustic attenuation chamber 7210 in a gap 7220 between the inner tube portion 7140 and the outer tube portion 7130. The acoustic attenuation chamber 7210 may be a region of the gap 7220 having a larger cross-sectional area than adjacent regions of the gap 7220. The acoustic attenuation chamber 7210 is arranged to, in use, reflect sound waves and attenuate the sound of a flow of air through the gap 7220. In the form of the technology shown in Figures 7A-7F, there are multiple acoustic attenuation chambers 7210 located at different positions circumferentially around the gap 7220 of the connection member 7000. The acoustic attenuation chamber 7210 may have the properties of a broad band muffler.

In some forms the noise attenuation structure 7200 comprises portions of the inner tube portion 7140 and/or the outer tube portion 7130 configured to form a stepped region 7230 in the gap 7220 between the inner tube portion 7140 and the outer tube portion 7130. The stepped region 7230 may be a region in which the flow path 6110 turns twice through approximately 90° turns, one turn being in the opposite direction to the other turn. The stepped region 7230 has the effect of reducing the kinetic energy of the flow of air flowing through it, resulting in a reduced velocity and less turbulent mixing, and therefore less noise generation, when the flow of air exits the gap 7220.

The stepped region 7230 may be located at the acoustic attenuation chamber 7210 such that the inlet to the acoustic attenuation chamber 7210 is offset from the outlet of the acoustic attenuation chamber 7210. Alternatively, the stepped region 7230 may be provided separate to, or instead of, an acoustic attenuation chamber 7210. In the form of the technology shown in Figures 7A-7F, the noise attenuation structure 7200 is located adjacent the end stop 7150. The stepped region 7230 may partially be formed by the corner portions of the end stop 7150.

### 5.6.3.6.1 Flow inhibitor

In the form of the technology where a connection member 7000 comprises the vent structure 6000, the connection member 7000 may comprise a flow inhibitor (not shown) to inhibit air inside the inner tube portion 7140 from exiting into the surrounding ambient air by flowing through the gap 7220 between the inner tube portion 7140 and the outer tube portion 7130. The flow inhibitor may reduce any noise generated by the flow of air through the gap 7220 between the inner tube portion 7140 and the outer tube portion 7130.

In one form, the flow inhibitor comprises an annular seal positioned to substantially block the flow of air through an opening to the gap 7220 between the inner tube portion 7140 and the outer tube portion 7130. For example, the annular seal may be positioned inside the tube portion 7100 abutting surfaces of the inner tube portion 7140 and the outer tube portion 7130 between which the opening to the gap 7220 is formed.

In some forms, the flow inhibitor may be formed from a flexible material, for instance a silicone or rubber. In some forms, the flow inhibitor may be friction fit or interference fit between the inner tube portion 7140 and the outer tube portion 7130. In other forms of the technology the flow inhibitor may comprise a snap fit connection between the inner tube portion 7140 and/or the outer tube portion 7130. In other forms of the technology the flow inhibitor may be adhered to or integrally formed with the inner tube portion 7140 and/or the outer tube portion 7130.

### 5.6.3.7 Curved airflow path

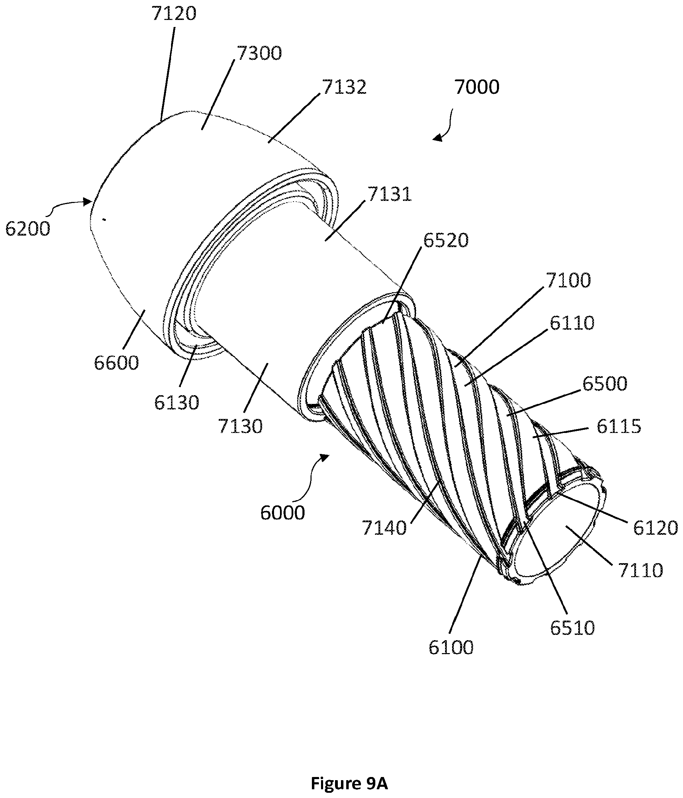

Another form of the technology in which the vent structure 6000 forms part of a connection member 7000 is illustrated in Figure 9A. The connection member 7000 is configured to directly or indirectly fluidly connect an air circuit 4170 to a patient interface 3000 and comprises several components including a tube portion 7100 and a vent structure 6000 for venting air from an interior volume of the tube portion 7100. The tube portion 7100 comprises a first end 7110 configured to directly or indirectly fluidly connect to the air circuit 4170 and a second end 7120 configured to directly or indirectly connect to the patient interface 3000. The tube portion also comprises an inner tube portion 7140 and an outer tube portion 7130. As in some forms of the technology described above, the vent structure comprises a vent housing 6100. The vent structure 6000 also comprises a plurality of partitions 6115 which form a plurality of flow paths 6110 therebetween.

The vent housing 6100 comprises at least a portion of the tube portion 7100. In the form of the technology shown in Figure 9A, substantially the full length of the tube portion 7100 houses the flow paths 6110, which are formed between the inner tube portion 7140 and the outer tube portion 7130. In other forms of the technology, the vent housing 6100 comprises only a portion of the tube portion 7100, for instance the flow paths may extend along only part of the length of the tube portion 7100. In some forms of the technology the vent housing 6100 may comprise a central portion of the tube portion 7100, the end portions of the tube portion 7100 being configured to connect to other components in the respiratory therapy system, for instance the air circuit 4170 or patient interface 3000. The vent housing 6100 may comprise one or more additional components which, together with a portion of the tube portion 7100, form the vent housing 6100.

In the form of the technology shown in Figure 9A the partitions 6115 are formed as part of the inner tube portion 7140.. In other forms of the technology, the partitions may be formed on an inner surface of the outer tube portion 7130. In other forms of the technology, the partitions may be formed as part of one or more separate components that are positioned between the inner tube portion 7140 and outer tube portion 7130 to form the flow paths 6110.

In the form of the technology shown in Figure 9A the partitions are distributed evenly around tube portion 7100.

As described above, each of the flow paths 6110 comprises a vent inlet 6120 configured to receive an air flow and a vent outlet 6130 configured to allow the air flow to exit into the surrounding ambient air. In the form shown in Figure 9A, each vent inlet 6120 is formed as a space between the inner tube portion 7140, the outer tube portion 7130 and two of the partitions 6115, and each inlet is configured to receive air from inside the tube portion 7100. In other forms of the technology, the vent inlet 6120 may be formed entirely by the inner tube portion 7140, for example as an opening in the inner tube portion 7140. In Figure 9A, the vent inlet 6120 is located at the first end 7110 of the tube portion 7100. In other forms, the vent inlet 6120 may be formed at the second end 7120 of the tube portion 7100. In still other forms, the vent inlet 6120 may be formed at an intermediate region of the tube portion 7100 between the first and second ends. In the embodiment shown in Figure 9A, the vent outlet 6130 is located at the opposite end of the flow path 6110 to the vent inlet 6120 and, as described below, is formed by the outer tube portion 7130. In other forms of the technology, the vent outlet 6130 may be located between the inner tube portion 7140 and the outer tube portion 7130 and at an opposite end of the tube portion 7100 to the vent inlet 6120.

In forms of the technology, a portion of the flow paths 6110 are curved around the tube portion 7100, i.e. the flow paths 6110 follow a curved path across the generally cylindrical surface of a part of the tube portion 7100, e.g. across the outer surface of the inner tube portion 7140. By having a portion of the flow paths 6110 curving around the tube portion 7100 the length of the flow paths 6110 is increased compared to flow paths having the form of straight paths in the longitudinal direction along the outer surface of tube portion 7100, without significantly increasing the length of the connection member 7000 in order to achieve that length. The advantages of increasing the length of the flow paths 6110 are discussed above in detail but generally it assists in reducing the noise produced by venting air.

In Figure 9A the plurality of partitions 6115 are configured so that a first portion 6500 of the flow paths 6110 are curved around the circumference of the inner tube portion 7140 and also extend longitudinally along the length of the inner tube portion 7140 (but at an angle to the longitudinal axis of the tube portions). The first portion 6500 of the flow path 6110 may have two ends 6510, 6520, the first end 6510 at the vent inlet 6120 and the second end 6520 located prior to a turning region, which is described below. In other forms of the technology, the vent outlet 6130 may be located at the second end 6520, i.e. the flow path 6110 may comprise only the first portion 6500 and not include the turning region. Each of the first portions 6500 of the flow paths 6110 are curved in the same direction and follow a similar shape. This ensures the flow paths 6110 remain separate and do not coincide with adjacent flow paths 6110. The first portions 6500 of the flow paths therefore may maintain a substantially constant distance between the partitions 6115 along the entire length of the flow paths 6110. This may also be described as the flow paths 6110 or the partitions 6115 having a constant width. In Figure 9A, the first portions 6500 form a spiral, or substantially spiral, shape around the tube portion 7100. The flow paths 6110 curve in a clockwise direction around the tube portion 7100 when viewed from the first end 7110. In other forms of the technology, the flow paths 6110 may curve in the opposite direction, i.e. the anti-clockwise direction when viewed from the first end 7110, or form another shape around the tube portion 7100 to that shown in Figure 9A, for instance a zig-zag shape, serpentine shape, or other convoluted shape which results in an increased length of the flow paths 6110 along the tube portion 7100.

In Figure 9A, the ends 6510, 6520 of the first portion 6500 of each flow path 6110 are circumferentially offset from each other around the tube portion 7100 due to the shape of the flow path 6110 around the tube portion 7100. In other words the ends 6510, 6520 are located at different positions around the circumference of the tube portion 7100 because of the curve in the flow path 6110. In other forms of the technology, where the vent outlet 6130 is located at the second end 6520, this results in the vent inlet 6120 and the vent outlet 6130 being circumferentially offset around the tube portion 7100. In some forms of the technology, the first portions 6500 of the flow paths 6110 may curve around the tube portion 7100 such that the ends 6510, 6520 are circumferentially aligned. In other words, the ends 6510, 6520 are located at the same position around the circumference of the tube portion 7100.

In certain forms (including the form illustrated in Figure 9A), the connection member 7000 may also include a turning region 6200 to change the direction of air flow to a substantially opposite direction and to further lengthen the flow paths 6110, as described above. The turning region 6200 may include one or more of the various features discussed above in sections 4.6.3.2 ('Shape of flow paths') and 4.6.3.3 ('Width of the flow path').

The turning region 6200 may be located downstream of the first portion 6500 of the flow path 6110. In the form shown in Figure 9A, there are no partitions 6115 downstream of the first portion 6500, and this results in the second portions 6600 of the flow paths 6110 joining to form a single flow path for that part of their length. In other forms of the technology, the partitions 6115 may continue along the entire length of the flow path 6110, defining both the first and second portions of the flow paths, from the vent inlet 6120 to the vent outlet 6130.

In the form of technology shown in Figure 9A, the turning region 6200 is formed entirely by the outer tube portion 7130 which curves at one end to form a turning portion 7300. In some forms, the turning portion 7300 has a convex outer surface that extends around an end of the tube portion 7100. The convex outer surface may extend around the outer circumference of the tube portion 7100. The convex outer surface may have a greater curvature closer to the second end 7120. The turning portion 7300 may be partially dome-shaped, mushroom-shaped, or umbrella-shaped. In Figure 9A, the outer tube portion 7130 curves away from the central longitudinal axis of the tube portion 7100 making a U-shaped, or substantially 180°, turn. The form of the outer tube portion 7130 is configured to substantially reverse the flow of air. In the first flow portion 6500 the flow of air is generally flowing in a direction from the first end 7110 to the second end 7120. After the turning region 6200 the flow of air is flowing generally in a direction from the second end 7120 to the first end 7110. In other forms of the technology, the turning region 6200 may change the direction of the flow of air by a different angle between 90° and 180°, as discussed above in discussed in section 4.6.3.2 ('Shape of flow paths').

In Figure 9A, a first region 7131 of the outer tube portion 7130 has a substantially hollow cylindrical shape. The hollow cylindrical shape is configured to fit around the inner tube portion 7140 and the first portions 6500 of the flow paths 6110 are formed therebetween. A second region 7132 of the outer tube portion 7130 is formed closer to the second end 7120 of the tube portion 7100 and comprises a curved flange that extends outwardly from the first region 7131. The second region 7132 comprises a surface which extends back over the outside surface of the first region 7131 and one or more flow paths 6110 are formed between the inner surface of the second region 7132 and the outer surface of the first region 7131. The second region 7132 also forms a substantially hollow cylindrical shape. In Figure 9A, the second region 7132 has a smaller radius at the second end 7120 of the tube portion 7100 compared to the end forming the vent outlet 6130. The second region 7132 forms a surface which has a smooth curve between its opposite ends, i.e. from the second end 7120 to the vent outlet 6130. This smooth curve helps to direct the flow of air exiting the vent towards the outer surface of the first region 7131 which assists in reducing noise for the reasons discussed above. In other forms of the technology the second region 7132 may have a different shape, for instance a shape that doesn't have an increasing radius, or that has a radius that increases and then decreases again between the second end 7120 and the vent outlet 6130.

In other forms of the technology the turning region 6200 may be formed between an inner surface of a flange 7145 comprised as part of the inner tube portion 7140 and an outer surface of the outer tube portion 7130, as discussed in section 4.6.3.2 ('Shape of flow paths').

### 5.7 HUMIDIFIER

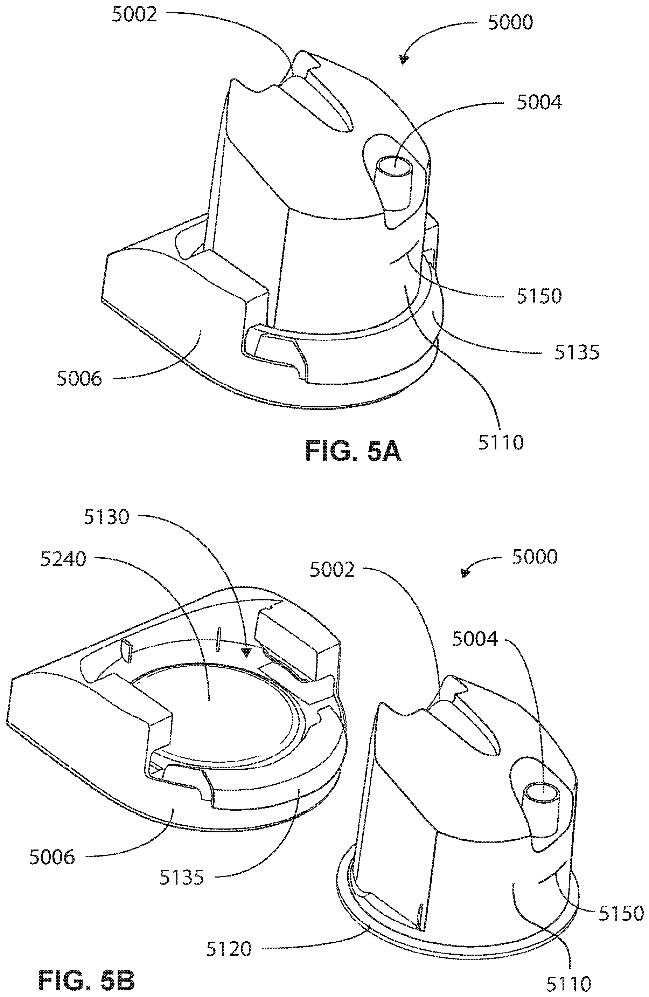

In one form of the present technology there is provided a humidifier 5000 (e.g. as shown in Fig. 5A) to change the absolute humidity of air or gas for delivery to a patient relative to ambient air. Typically, the humidifier 5000 is used to increase the absolute humidity and increase the temperature of the flow of air (relative to ambient air) before delivery to the patient's airways.

The humidifier 5000 may comprise a humidifier reservoir 5110, a humidifier inlet 5002 to receive a flow of air, and a humidifier outlet 5004 to deliver a humidified flow of *air.* In some forms, as shown in Fig. 5A and Fig. 5B, an inlet and an outlet of the humidifier reservoir 5110 may be the humidifier inlet 5002 and the humidifier outlet 5004 respectively. The humidifier 5000 may further comprise a humidifier base 5006, which may be adapted to receive the humidifier reservoir 5110 and comprise a heating element 5240.

### 5.8 GLOSSARY

For the purposes of the present technology disclosure, in certain forms of the present technology, one or more of the following definitions may apply. In other forms of the present technology, alternative definitions may apply.

### 5.8.1 General

*Air:* In certain forms of the present technology, air may be taken to mean atmospheric air, and in other forms of the present technology air may be taken to mean some other combination of breathable gases, e.g. atmospheric air enriched with oxygen.

*Ambient:* In certain forms of the present technology, the term ambient will be taken to mean (i) external of the treatment system or patient, and (ii) immediately surrounding the treatment system or patient.

For example, ambient humidity with respect to a humidifier may be the humidity of air immediately surrounding the humidifier, e.g. the humidity in the room where a patient is sleeping. Such ambient humidity may be different to the humidity outside the room where a patient is sleeping.

In another example, ambient pressure may be the pressure immediately surrounding or external to the body.

In certain forms, ambient (e.g., acoustic) noise may be considered to be the background noise level in the room where a patient is located, other than for example, noise generated by an RPT device or emanating from a mask or patient interface. Ambient noise may be generated by sources outside the room.

*Automatic Positive Airway Pressure (APAP) therapy:* CPAP therapy in which the treatment pressure is automatically adjustable, e.g. from breath to breath, between minimum and maximum limits, depending on the presence or absence of indications of SDB events.

*Continuous Positive Airway Pressure (CPAP) therapy:* Respiratory pressure therapy in which the treatment pressure is approximately constant through a respiratory cycle of a patient. In some forms, the pressure at the entrance to the airways will be slightly higher during exhalation, and slightly lower during inhalation. In some forms, the pressure will vary between different respiratory cycles of the patient, for example, being increased in response to detection of indications of partial upper airway obstruction, and decreased in the absence of indications of partial upper airway obstruction.

*Flow rate:* The volume (or mass) of air delivered per unit time. Flow rate may refer to an instantaneous quantity. In some cases, a reference to flow rate will be a reference to a scalar quantity, namely a quantity having magnitude only. In other cases, a reference to flow rate will be a reference to a vector quantity, namely a quantity having both magnitude and direction. Flow rate may be given the symbol Q. 'Flow rate' is sometimes shortened to simply 'flow' or 'airflow'.

In the example of patient respiration, a flow rate may be nominally positive for the inspiratory portion of a breathing cycle of a patient, and hence negative for the expiratory portion of the breathing cycle of a patient. Device flow rate, Qd, is the flow rate of air leaving the RPT device. Total flow rate, Qt, is the flow rate of air and any supplementary gas reaching the patient interface via the air circuit. Vent flow rate, *Qv,* is the flow rate of air leaving a vent to allow washout of exhaled gases. Leak flow rate, Ql, is the flow rate of leak from a patient interface system or elsewhere. Respiratory flow rate, Qr, is the flow rate of air that is received into the patient's respiratory system.

*Flow therapy:* Respiratory therapy comprising the delivery of a flow of air to an entrance to the airways at a controlled flow rate referred to as the treatment flow rate that is typically positive throughout the patient's breathing cycle.

*Humidifier:* The word humidifier will be taken to mean a humidifying apparatus constructed and arranged, or configured with a physical structure to be capable of providing a therapeutically beneficial amount of water (H₂O) vapour to a flow of air to ameliorate a medical respiratory condition of a patient.

*Leak:* The word leak will be taken to be an unintended flow of air. In one example, leak may occur as the result of an incomplete seal between a mask and a patient's face. In another example leak may occur in a swivel elbow to the ambient.

*Noise, conducted* (*acoustic*): Conducted noise in the present document refers to noise which is carried to the patient by the pneumatic path, such as the air circuit and the patient interface as well as the air therein. In one form, conducted noise may be quantified by measuring sound pressure levels at the end of an air circuit.

*Noise, radiated (acoustic):* Radiated noise in the present document refers to noise which is carried to the patient by the ambient air. In one form, radiated noise may be quantified by measuring sound power/pressure levels of the object in question according to ISO 3744.

*Noise, vent (acoustic):* Vent noise in the present document refers to noise which is generated by the flow of air through any vents such as vent holes of the patient interface.

*Patient:* A person, whether or not they are suffering from a respiratory condition.

*Pressure:* Force per unit area. Pressure may be expressed in a range of units, including cmH₂O, g-f/cm² and hectopascal. 1 cmH₂O is equal to 1 g-f/cm² and is approximately 0.98 hectopascal (1 hectopascal = 100 Pa = 100 N/m² = 1 millibar ~ 0.001 atm). In this specification, unless otherwise stated, pressure is given in units of cmH₂O.

The pressure in the patient interface is given the symbol Pm, while the treatment pressure, which represents a target value to be achieved by the interface pressure *Pm* at the current instant of time, is given the symbol *Pt.*

*Respiratory Pressure Therapy (RPT):* The application of a supply of air to an entrance to the airways at a treatment pressure that is typically positive with respect to atmosphere.

*Ventilator:* A mechanical device that provides pressure support to a patient to perform some or all of the work of breathing.

### 5.8.1.1 Materials

*Silicone or Silicone Elastomer:* A synthetic rubber. In this specification, a reference to silicone is a reference to liquid silicone rubber (LSR) or a compression moulded silicone rubber (CMSR). One form of commercially available LSR is SILASTIC (included in the range of products sold under this trademark), manufactured by Dow Corning. Another manufacturer of LSR is Wacker. Unless otherwise specified to the contrary, an exemplary form of LSR has a Shore A (or Type A) indentation hardness in the range of about 35 to about 45 as measured using ASTM D2240. (Year? Required?)

*Polycarbonate:* a thermoplastic polymer of Bisphenol-A Carbonate.

### 5.8.1.2 Mechanical properties

*Resilience:* Ability of a material to absorb energy when deformed elastically and to release the energy upon unloading.

*Resilient:* Will release substantially all of the energy when unloaded. Includes e.g. certain silicones, and thermoplastic elastomers.

*Hardness:* The ability of a material *per seto* resist deformation (e.g. described by a Young's Modulus, or an indentation *hardness* scale measured on a standardised sample size).
- 'Soft' materials may include silicone or thermo-plastic elastomer (TPE), and may, e.g. readily deform under finger pressure.
- 'Hard' materials may include polycarbonate, polypropylene, steel or aluminium, and may not e.g. readily deform under finger pressure.

*Stiffness (or rigidity) of a structure or component:* The ability of the structure or component to resist deformation in response to an applied load. The load may be a force or a moment, e.g. compression, tension, bending or torsion. The structure or component may offer different resistances in different directions. The inverse of stiffness is *flexibility.*

*Floppy structure or component:* A structure or component that will change shape, e.g. bend, when caused to support its own weight, within a relatively short period of time such as 1 second.

*Rigid structure or component:* A structure or component that will not substantially change shape when subject to the loads typically encountered in use. An example of such a use may be setting up and maintaining a patient interface in sealing relationship with an entrance to a patient's airways, e.g. at a load of approximately 20 to 30 cmH₂O pressure.

As an example, an I-beam may comprise a different bending stiffness (resistance to a bending load) in a first direction in comparison to a second, orthogonal direction. In another example, a structure or component may be floppy in a first direction and rigid in a second direction.

### 5.8.2 Patient interface

*Anti-asphyxia value (AAV):* The component or sub-assembly of a mask system that, by opening to atmosphere in a failsafe manner, reduces the risk of excessive CO₂ rebreathing by a patient.

*Elbow:* An elbow is an example of a structure that directs an axis of flow of air travelling therethrough to change direction through an angle. In one form, the angle may be approximately 90 degrees. In another form, the angle may be more, or less than 90 degrees. The elbow may have an approximately circular cross-section. In another form the elbow may have an oval or a rectangular cross-section. In certain forms an elbow may be rotatable with respect to a mating component, e.g. about 360 degrees. In certain forms an elbow may be removable from a mating component, e.g. via a snap connection. In certain forms, an elbow may be assembled to a mating component via a one-time snap during manufacture, but not removable by a patient.

*Frame:* Frame will be taken to mean a mask structure that bears the load of tension between two or more points of connection with a headgear. A mask frame may be a non-airtight load bearing structure in the mask. However, some forms of mask frame may also be air-tight.

*Headgear:* Headgear will be taken to mean a form of positioning and stabilizing structure designed for use on a head. For example the headgear may comprise a collection of one or more struts, ties and stiffeners configured to locate and retain a patient interface in position on a patient's face for delivery of respiratory therapy. Some ties are formed of a soft, flexible, elastic material such as a laminated composite of foam and fabric.

*Membrane:* Membrane will be taken to mean a typically thin element that has, preferably, substantially no resistance to bending, but has resistance to being stretched.

*Plenum chamber:* a mask plenum chamber will be taken to mean a portion of a patient interface having walls at least partially enclosing a volume of space, the volume having air therein pressurised above atmospheric pressure in use. A shell may form part of the walls of a mask plenum chamber.

*Seal:* May be a noun form ("a seal") which refers to a structure, or a verb form ("to seal") which refers to the effect. Two elements may be constructed and/or arranged to 'seal' or to effect 'sealing' therebetween without requiring a separate 'seal' element *per se.*

*Shell:* A shell will be taken to mean a curved, relatively thin structure having bending, tensile and compressive stiffness. For example, a curved structural wall of a mask may be a shell. In some forms, a shell may be faceted. In some forms a shell may be airtight. In some forms a shell may not be airtight.

*Stiffener:* A stiffener will be taken to mean a structural component designed to increase the bending resistance of another component in at least one direction.

*Strut:* A strut will be taken to be a structural component designed to increase the compression resistance of another component in at least one direction.

*Swivel* (noun): A subassembly of components configured to rotate about a common axis, preferably independently, preferably under low torque. In one form, the swivel may be constructed to rotate through an angle of at least 360 degrees. In another form, the swivel may be constructed to rotate through an angle less than 360 degrees. When used in the context of an air delivery conduit, the sub-assembly of components preferably comprises a matched pair of cylindrical conduits. There may be little or no leak flow of air from the swivel in use.

*Tie* (noun): A structure designed to resist tension.

*Vent:* (noun): A structure that allows a flow of *air* from an interior of the mask, or conduit, to ambient air for clinically effective washout of exhaled gases. For example, a clinically effective washout may involve a flow rate of about 10 litres per minute to about 100 litres per minute, depending on the mask design and treatment pressure.

### 5.9 OTHER REMARKS

A portion of the disclosure of this patent document contains material which is subject to copyright protection. The copyright owner has no objection to the facsimile reproduction by anyone of the patent document or the patent disclosure, as it appears in Patent Office patent files or records, but otherwise reserves all copyright rights whatsoever.

Unless the context clearly dictates otherwise and where a range of values is provided, it is understood that each intervening value, to the tenth of the unit of the lower limit, between the upper and lower limit of that range, and any other stated or intervening value in that stated range is encompassed within the technology. The upper and lower limits of these intervening ranges, which may be independently included in the intervening ranges, are also encompassed within the technology, subject to any specifically excluded limit in the stated range. Where the stated range includes one or both of the limits, ranges excluding either or both of those included limits are also included in the technology.

Furthermore, where a value or values are stated herein as being implemented as part of the technology, it is understood that such values may be approximated, unless otherwise stated, and such values may be utilized to any suitable significant digit to the extent that a practical technical implementation may permit or require it.

Unless defined otherwise, all technical and scientific terms used herein have the same meaning as commonly understood by one of ordinary skill in the art to which this technology belongs. Although any methods and materials similar or equivalent to those described herein can also be used in the practice or testing of the present technology, a limited number of the exemplary methods and materials are described herein.

When a particular material is identified as being used to construct a component, obvious alternative materials with similar properties may be used as a substitute. Furthermore, unless specified to the contrary, any and all components herein described are understood to be capable of being manufactured and, as such, may be manufactured together or separately.

It must be noted that as used herein and in the appended claims, the singular forms "a", "an", and "the" include their plural equivalents, unless the context clearly dictates otherwise.

The publications discussed herein are provided solely for their disclosure prior to the filing date of the present application. Nothing herein is to be construed as an admission that the present technology is not entitled to antedate such publication by virtue of prior invention. Further, the dates of publication provided may be different from the actual publication dates, which may need to be independently confirmed.

The terms "comprises" and "comprising" should be interpreted as referring to elements, components, or steps in a non-exclusive manner, indicating that the referenced elements, components, or steps may be present, or utilized, or combined with other elements, components, or steps that are not expressly referenced.

The subject headings used in the detailed description are included only for the ease of reference of the reader and should not be used to limit the subject matter found throughout the disclosure or the claims. The subject headings should not be used in construing the scope of the claims or the claim limitations.

Although the technology herein has been described with reference to particular examples, it is to be understood that these examples are merely illustrative of the principles and applications of the technology. In some instances, the terminology and symbols may imply specific details that are not required to practice the technology. For example, although the terms "first" and "second" may be used, unless otherwise specified, they are not intended to indicate any order but may be utilised to distinguish between distinct elements. Furthermore, although process steps in the methodologies may be described or illustrated in an order, such an ordering is not required. Those skilled in the art will recognize that such ordering may be modified and/or aspects thereof may be conducted concurrently or even synchronously.

It is therefore to be understood that numerous modifications may be made to the illustrative examples and that other arrangements may be devised without departing from the scope of the technology.

### 5.10 REFERENCE SIGNS LIST

- 1000: Patient
- 1100: Bed partner
- 3000: Patient interface
- 3100: Seal-forming structure
- 3200: Plenum chamber
- 3300: Positioning and stabilising structure
- 3400: Vent
- 3600: Connection port
- 3700: Forehead support
- 3800: Cannula
- 3810a: Nasal prongs
- 3810b: Nasal prongs
- 3820a: Lumens
- 3820b: Lumens
- 4000: RPT device
- 4010: External housing
- 4012: Upper portion
- 4014: Lower portion
- 4015: Panel(s)
- 4016: Chassis
- 4018: Handle
- 4020: Pneumatic block
- 4100: Pneumatic components
- 4110: Air filters
- 4112: Inlet air filter
- 4114: Outlet air filter
- 4120: Muffler
- 4122: Inlet muffler
- 4124: Outlet muffler
- 4140: Pressure generator
- 4142: Blower
- 4144: Brushless DC motor
- 4160: Anti-spill back valve
- 4170: Air circuit
- 4180: Supplemental oxygen
- 4200: Electrical components
- 4202: Printed Circuit Board Assembly (PCBA)
- 4210: Power Supply
- 4220: Input devices
- 4270: Transducers
- 5000: Humidifier
- 5002: Humidifier inlet
- 5004: Humidifier outlet
- 5006: Humidifier base
- 5110: Reservoir
- 5120: Conductive portion
- 5130: Humidifier reservoir dock
- 5135: Locking lever
- 5150: Water level indicator
- 5240: Heating element
- 6000: Vent structure
- 6100: Vent housing
- 6102: First end of vent housing
- 6104: Second end of vent housing
- 6106: Connector
- 6110: Flow path
- 6115: Partitions
- 6120: Vent inlet
- 6130: Vent outlet
- 6200: Turning region
- 6210: Portion of the turning region
- 6300: Inner path surface
- 6310: Flat region
- 6320: Curved region
- 6330: First point
- 6340: Second point
- 6350: Region of maximum thickness
- 6400: Outer path surface
- 6500: First portion
- 6510: First end of the first portion
- 6520: Second end of the first portion
- 6600: Second portion
- 7000: Connection member
- 7100: Tube portion
- 7110: First end
- 7120: Second end
- 7130: Outer tube portion
- 7131: First region of the outer tube portion
- 7132: Second region of the outer tube portion
- 7140: Inner tube portion
- 7145: Flange
- 7150: End stop
- 7200: Noise attenuation structure
- 7210: Acoustic attenuation chamber
- 7220: Gap
- 7230: Stepped region
- 7300: Turning portion

## Claims

1. A vent structure (6000) for a respiratory therapy system, the vent structure (6000) comprising:
a vent housing (6100) defining:
a flow path (6110) for a flow of air being vented from the respiratory therapy system;
a vent inlet (6120) configured to allow the flow of air to enter the flow path (6110); and
a vent outlet (6130) configured to allow the flow of air to exit the flow path into the surrounding ambient air;
wherein the vent housing (6100) is configured to comprise a curved turning region (6200) defining a curved flow path (6110) in which the flow path (6110) changes direction by substantially 180°, and wherein the vent housing (6100) is configured to comprise an inner path surface (6300) on an inner side of the curved flow path (6110), and an outer path surface (6400) on an outer side of the curved flow path (6110);
wherein the width of the curved flow path (6110) at the curved turning region (6200) and at a region downstream of the curved turning region (6200) is at least substantially 0.85mm; and
wherein an opening angle between the inner path surface of the vent housing (6100) at the vent outlet (6130) and the outer path surface of the vent housing (6100) at the vent outlet (6130) is substantially 7° or less.

2. The vent structure (6000) according to claim 1, wherein, in the curved turning region (6110), the inner path surface has a cross-sectional shape in the form of a segment of a circle.

3. The vent structure (6000) according to claim 1 or 2, wherein through the curved turning region (6200) the flow path (6110) changes direction to a substantially opposite direction.

4. The vent structure (6000) according to any one of claims 1 to 3, wherein the inner path surface has a cross-sectional shape in, or resembling, the form of part of an aerofoil.

5. The vent structure (6000) according to any one of claims 1 to 4, wherein the inner path surface (6300) defines a chord line having a chord length of at least 50mm.

6. The vent structure (6000) according to claim 5, wherein a region of maximum thickness of the inner path surface (6300) is located along the chord line at a distance from an end of the chord line proximate the turning region (6200) that is at least 25% of the chord length.

7. The vent structure (6000) according to claim 6, wherein the vent outlet (6130) is located downstream of the region of maximum thickness.

8. The vent structure (6000) according to any one of claims 1 to 7, wherein the vent housing (6100) comprises a plurality of partitions which form a plurality of flow paths therebetween, wherein the plurality of flow paths comprise the flow path and the other flow paths of the plurality of flow paths are similar to the flow path.

9. A patient interface (3000) comprising:
a plenum chamber (3200) pressurisable to a therapeutic pressure of at least 6 cmH₂O above ambient air pressure, said plenum chamber (3200) including a plenum chamber inlet port sized and structured to receive a flow of air at the therapeutic pressure for breathing by a patient (1000),
a seal-forming structure (3100) constructed and arranged to form a seal with a region of the patient's face surrounding an entrance to the patient's airways, said seal-forming structure (3100) having a hole therein such that the flow of air at said therapeutic pressure is delivered to at least an entrance to the patient's nares, the seal-forming structure (3100) constructed and arranged to maintain said therapeutic pressure in the plenum chamber (3200) throughout the patient's respiratory cycle in use; and
a vent structure (6000) according to any one of claims 1 to 8, wherein the vent structure (6000) allows a continuous flow of gases exhaled by the patient (1000) from an interior of the plenum chamber (3200) to ambient, said vent structure (6000) being sized and shaped to maintain the therapeutic pressure in the plenum chamber (3200) in use,
wherein the patient interface (3000) is configured to allow the patient (1000) to breathe from ambient through their mouth in the absence of a flow of pressurised air through the plenum chamber inlet port, or the patient interface is a nasal mask configured to leave the patient's mouth uncovered.

10. A connection member (7000) configured to directly or indirectly fluidly connect an air circuit (4170) to a patient interface in a respiratory therapy system, the connection member (7000) comprising:
a tube portion (7100) comprising a first end (7110) configured to directly or indirectly fluidly connect to the air circuit and a second end (7120) configured to directly or indirectly fluidly connect to the patient interface (3000); and
a vent structure (6000) according to any one of claims 1 to 8, the vent structure (6000) being configured to allow air in the tube portion (7100) to exit into the surrounding ambient air.

11. The connection member of claim 10, wherein the tube portion (7100) comprises:
an outer tube portion (7130); and
an inner tube (7140) portion.

12. The connection member (7000) of claim 11, wherein the outer tube portion (7130) is rotatable relative to the inner tube portion (7140) around a mutual longitudinal axis.

13. The connection member (7000) of any one of claims 11 or 12, wherein the outer tube portion (7130) comprises the first end (7110) and the inner tube portion (7140) comprises the second end (7120).

## Patentansprüche

1. Entlüftungsstruktur (6000) für ein Atemtherapiesystem, wobei die Entlüftungsstruktur (6000) aufweist:
ein Entlüftungsgehäuse (6100), das definiert:
einen Strömungspfad (6110) für einen aus dem Atemtherapiesystem ausströmenden Luftstrom;
einen Entlüftungseinlass (6120), der konfiguriert ist, dem Luftstrom zu erlauben, in den Strömungspfad (6110) einzutreten; und
einen Entlüftungsauslass (6130), der konfiguriert ist, dem Luftstrom zu erlauben, aus dem Strömungspfad in die umgebende Außenluft auszutreten;
wobei das Entlüftungsgehäuse (6100) konfiguriert ist, eine gekrümmte Wendezone (6200) aufzuweisen, die einen gekrümmten Strömungspfad (6110) definiert, in dem der Strömungspfad (6110) seine Richtung um im Wesentlichen 180° ändert,
und wobei das Entlüftungsgehäuse (6100) konfiguriert ist, eine innere Pfadfläche (6300) an einer Innenseite des gekrümmten Strömungspfads (6110) und eine äußere Pfadfläche (6400) an einer Außenseite des gekrümmten Strömungspfads (6110) aufzuweisen;
wobei die Breite des gekrümmten Strömungspfads (6110) an der gekrümmten Wendezone (6200) und an einer Zone stromabwärts der gekrümmten Wendezone (6200) mindestens im Wesentlichen 0,85mm ist; und
wobei ein Öffnungswinkel zwischen der inneren Pfadfläche des Entlüftungsgehäuses (6100) an dem Entlüftungsauslass (6130) und der äußeren Pfadfläche des Entlüftungsgehäuses (6100) an dem Entlüftungsauslass (6130) im Wesentlichen 7° oder weniger ist.

2. Entlüftungsstruktur (6000) nach Anspruch 1, wobei in der gekrümmten Wendezone (6110) die innere Pfadfläche ein Querschnittsprofil in der Form eines Segments eines Kreises hat.

3. Entlüftungsstruktur (6000) nach Anspruch 1 oder 2, wobei durch die gekrümmte Wendezone (6200) der Strömungspfad (6110) seine Richtung in eine im Wesentlichen entgegengesetzte Richtung ändert.

4. Entlüftungsstruktur (6000) nach einem der Ansprüche 1 bis 3, wobei die innere Pfadfläche ein Querschnittsprofil in oder ähnlich der Form eines Teils eines Tragflügels hat.

5. Entlüftungsstruktur (6000) nach einem der Ansprüche 1 bis 4, wobei die innere Pfadfläche (6300) eine Sehnenlinie mit einer Sehnenlänge von mindestens 50mm definiert.

6. Entlüftungsstruktur (6000) nach Anspruch 5, wobei eine Zone maximaler Dicke der inneren Pfadfläche (6300) sich entlang der Sehnenlinie in einem Abstand von einem der Wendezone (6200) nächstgelegenen Ende der Sehnenlinie befindet, der mindestens 25% der Sehnenlinie ist.

7. Entlüftungsstruktur (6000) nach Anspruch 6, wobei der Entlüftungsauslass (6130) sich stromabwärts der Zone maximaler Dicke befindet.

8. Entlüftungsstruktur (6000) nach einem der Ansprüche 1 bis 7, wobei das Entlüftungsgehäuse (6100) mehrere Zwischenwände aufweist, die zwischen sich mehrere Strömungspfade bilden, wobei die mehreren Strömungspfade den Strömungspfad aufweisen und die anderen Strömungspfade der mehreren Strömungspfade dem Strömungspfad gleichen.

9. Patientenschnittstelle (3000) aufweisend:
eine Plenumkammer (3200), die auf einen therapeutischen Druck von mindestens 6 cmH₂O über Umgebungsluftdruck gebracht werden kann, wobei die Plenumkammer (3200) eine Plenumkammer-Einlassöffnung aufweist, die bemessen und strukturiert ist, einen von einem Patienten (1000) einzuatmenden Luftstrom mit dem therapeutischen Druck zu empfangen,
eine dichtungsbildende Struktur (3100), die konstruiert und eingerichtet ist, um mit einem Bereich des Patientengesichts, der einen Eingang zu den Luftwegen des Patienten umgibt, eine Dichtung zu bilden, wobei die dichtungsbildende Struktur (3100) ein Loch hat, so dass der Luftstrom mit dem therapeutischen Druck zumindest einem Eingang zu Nasenlöchern des Patienten zugeführt wird, wobei die dichtungsbildende Struktur (3100) konstruiert und eingerichtet ist, um in Gebrauch den therapeutischen Druck in der Plenumkammer (3200) über den gesamten Atmungszyklus des Patienten hinweg aufrechtzuerhalten; und
eine Entlüftungsstruktur (6000) nach einem der Ansprüche 1 bis 8, wobei die Entlüftungsstruktur (6000) einen kontinuierlichen Strom von Gasen, die von dem Patienten (1000) ausgeatmet werden, aus einem Inneren der Plenumkammer (3200) in die Umgebung erlaubt, wobei die Entlüftungsstruktur (6000) bemessen und gestaltet ist, um in Gebrauch den therapeutischen Druck in der Plenumkammer (3200) aufrechtzuerhalten,
wobei die Patientenschnittstelle (300) konfiguriert ist, bei Abwesenheit eines Druckluftstroms durch die Plenumkammer-Einlassöffnung dem Patienten (1000) zu erlauben, von außen durch den Mund einzuatmen, oder die Patientenschnittstelle eine Nasenmaske ist, die konfiguriert ist, den Mund des Patienten unbedeckt zu lassen.

10. Verbindungselement (7000), das konfiguriert ist, um in einem Atemtherapiesystem eine Luftleitung (4170) direkt oder indirekt mit einer Patientenschnittstelle fluid zu verbinden, wobei das Verbindungselement (7000) aufweist:
einen Rohrabschnitt (7100) mit einem ersten Ende (7110), das konfiguriert ist, direkt oder indirekt mit der Luftleitung fluid zu verbinden, und mit einem zweiten Ende (7120), das konfiguriert ist, direkt oder indirekt mit der Patientenschnittstelle (3000) fluid zu verbinden; und
eine Entlüftungsstruktur (6000) nach einem der Ansprüche 1 bis 8, wobei die Entlüftungsstruktur (6000) konfiguriert ist, Luft in dem Rohrabschnitt (7100) zu erlauben, in die umgebende Außenluft auszutreten.

11. Verbindungselement (7000) nach Anspruch 10, wobei der Rohrabschnitt (7100) aufweist:
einen äußeren Rohrabschnitt (7130); und
einen inneren Rohrabschnitt (7140).

12. Verbindungselement (7000) nach Anspruch 11, wobei der äußere Rohrabschnitt (7130) relativ zu dem inneren Rohrabschnitt (7140) um eine gemeinsame Längsachse rotierbar ist.

13. Verbindungselement (7000) nach einem der Ansprüche 11 oder 12, wobei der äußere Rohrabschnitt (7130) das erste Ende (7110) aufweist und der innere Rohrabschnitt (7140) das zweite Ende (7120) aufweist.

## Revendications

1. Structure d'évent (6000) destinée à un système de thérapie respiratoire, la structure d'évent (6000) comprenant :
un boîtier d'évent (6100) définissant :
un trajet d'écoulement (6110) pour un flux d'air qui est évacué du système de thérapie respiratoire ;
une entrée d'évent (6120) configurée pour permettre au flux d'air d'entrer dans le trajet d'écoulement (6110) ; et
une sortie d'évent (6130) configurée pour permettre au flux d'air de sortir du trajet d'écoulement dans l'air ambiant environnant ;
dans laquelle le boîtier d'évent (6100) est configuré pour comprendre une région de virage courbe (6200) définissant un trajet d'écoulement courbe (6110) dans laquelle le trajet d'écoulement (6110) change de direction de sensiblement 180°,
et dans laquelle le boîtier d'évent (6100) est configuré pour comprendre une surface de trajet intérieure (6300) d'un côté intérieur du trajet d'écoulement courbe (6110), et une surface de trajet extérieure (6400) d'un côté extérieur du trajet d'écoulement courbe (6110) ;
dans laquelle la largeur du trajet d'écoulement courbe (6110) au niveau de la région de virage courbe (6200) et au niveau d'une région en aval de la région de virage courbe (6200) est au moins sensiblement égale à 0,85 mm ; et
dans laquelle un angle d'ouverture entre la surface de trajet intérieure du boîtier d'évent (6100) au niveau de la sortie d'évent (6130) et la surface de trajet extérieure du boîtier d'évent (6100) au niveau de la sortie d'évent (6130) est sensiblement égale ou inférieure à 7°.

2. Structure d'évent (6000) selon la revendication 1, dans laquelle, dans la région de virage courbe (6110), la surface de trajet intérieure a une forme de section transversale sous la forme d'un segment d'un cercle.

3. Structure d'évent (6000) selon la revendication 1 ou la revendication 2, dans laquelle par l'intermédiaire de la région de virage courbe (6200), le trajet d'écoulement (6110) passe d'une direction à une direction sensiblement opposée.

4. Structure d'évent (6000) selon l'une quelconque des revendications 1 à 3, dans laquelle la surface de trajet intérieure a une section transversale sous la forme d'une partie d'un profil aérodynamique, ou y ressemblant.

5. Structure d'évent (6000) selon l'une quelconque des revendications 1 à 4, dans laquelle la surface de trajet intérieure (6300) définit une ligne de corde ayant une longueur de corde d'au moins 50 mm.

6. Structure d'évent (6000) selon la revendication 5, dans laquelle une région d'épaisseur maximale de la surface de trajet intérieure (6300) est située le long de la ligne de corde à une distance d'une extrémité de la ligne de corde proche de la région de virage (6200) qui est égale à au moins 25 % de la longueur de corde.

7. Structure d'évent (6000) selon la revendication 6, dans laquelle la sortie d'évent (6130) est située en aval de la région d'épaisseur maximale.

8. Structure d'évent (6000) selon l'une quelconque des revendications 1 à 7, dans laquelle le boîtier d'évent (6100) comprend une pluralité de cloisons qui forment, entre elles, une pluralité de trajets d'écoulement, dans laquelle les trajets de la pluralité de trajets d'écoulement comprennent le trajet d'écoulement et les autres trajets d'écoulement de la pluralité de trajets d'écoulement sont similaires au trajet d'écoulement.

9. Interface patient (3000), comprenant :
une chambre de distribution (3200) pouvant être mise sous pression à une pression thérapeutique d'au moins 6 cmH₂O au-dessus de la pression d'air ambiant, ladite chambre de distribution (3200) comprenant un orifice d'entrée de chambre de distribution dimensionné et structuré pour recevoir un flux d'air à la pression thérapeutique à des fins de respiration par un patient (1000),
une structure de formation d'étanchéité (3100) structurée et conçue pour former une étanchéité avec une région du visage du patient entourant une entrée des voies aériennes du patient, ladite structure de formation d'étanchéité (3100) comportant un trou en son sein de sorte que le flux d'air à ladite pression thérapeutique est délivré à au moins une entrée des narines du patient, la structure de formation d'étanchéité (3100) étant structurée et conçue pour maintenir ladite pression thérapeutique dans la chambre de distribution (3200) sur tout le cycle respiratoire du patient en utilisation ; et
une structure d'évent (6000) selon l'une quelconque des revendications 1 à 8, dans laquelle la structure d'évent (6000) permet un écoulement continu de gaz exhalés par le patient (1000) de l'intérieur de la chambre de distribution (3200) vers l'environnement ambiant, ladite structure d'évent (6000) étant dimensionnée et formée pour maintenir la pression thérapeutique dans la chambre de distribution (3200) en utilisation,
dans laquelle l'interface patient (3000) est configurée pour permettre au patient (1000) de respirer par la bouche à partir de l'environnement ambiant en l'absence d'un flux d'air sous pression à travers l'orifice d'entrée de chambre de distribution, ou l'interface patient est un masque nasal configuré pour laisser non couverte la bouche du patient.

10. Élément de connexion (7000) configuré pour connecter directement ou indirectement en communication fluidique un circuit d'air (4170) à une interface patient d'un système de thérapie respiratoire, l'élément de connexion (7000) comprenant :
une partie tube (7100) comprenant une première extrémité (7110) configurée pour être connectée directement ou indirectement en communication fluidique au circuit d'air et une seconde extrémité (7120) configurée pour être connectée directement ou indirectement en communication fluidique à l'interface patient (3000) ; et
une structure d'évent (6000) selon l'une quelconque des revendications 1 à 8, la structure d'évent (6000) étant configurée pour permettre à de l'air se trouvant dans la partie tube (7100) de sortir dans l'air ambiant environnant.

11. Élément de connexion (7000) selon la revendication 10, dans lequel la partie tube (7100) comprend :
une partie de tube extérieur (7130) ; et
une partie de tube intérieur (7140).

12. Élément de connexion (7000) selon la revendication 11, dans lequel la partie de tube extérieur (7130) est mobile en rotation par rapport à la partie de tube intérieur (7140) autour d'un axe longitudinal commun.

13. Élément de connexion (7000) selon l'une ou l'autre des revendications 11 et 12, dans lequel la partie de tube extérieur (7130) comprend la première extrémité (7110) et la partie de tube intérieur (7140) comprend la seconde extrémité (7120).
